(19)

Europäisches Patentamt
European Patent Office
Office européen des brevets

(11) **EP 3 275 376 A1**

(12) **EUROPEAN PATENT APPLICATION**
published in accordance with Art. 153(4) EPC

(43) Date of publication:
**31.01.2018 Bulletin 2018/05**

(21) Application number: **15886503.0**

(22) Date of filing: **02.12.2015**

(51) Int Cl.:
**A61B 8/14** (2006.01)

(86) International application number:
**PCT/JP2015/083929**

(87) International publication number:
**WO 2016/151951 (29.09.2016 Gazette 2016/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **23.03.2015 JP 2015059977**

(71) Applicant: **Olympus Corporation**
**Hachioji-shi**
**Tokyo 192-8507 (JP)**

(72) Inventor: **ICHIKAWA, Junichi**
**Tokyo 192-8507 (JP)**

(74) Representative: **Gunzelmann, Rainer**
**Wuesthoff & Wuesthoff**
**Patentanwälte PartG mbB**
**Schweigerstraße 2**
**81541 München (DE)**

(54) **ULTRASONIC OBSERVATION DEVICE, ULTRASONIC OBSERVATION DEVICE OPERATION METHOD, AND ULTRASONIC OBSERVATION DEVICE OPERATION PROGRAM**

(57)      An ultrasound observation apparatus according to the present invention includes: a frequency analysis unit that analyzes the frequency of an ultrasound signal to calculate a plurality of frequency spectra in accordance with the reception depths and receiving directions of the ultrasound signal; a feature calculation unit that calculates a feature of each frequency spectrum; an attenuation factor setting unit that sets an attenuation factor of a computation-purpose region being a region that is different from a region of interest in an ultrasound image and is used for computation for correcting the feature; and a feature correction unit that performs attenuation correction on the feature using the attenuation factor set by the attenuation factor setting unit to calculate a corrected feature at a sampling point in the region of interest in the ultrasound image.

FIG.1

EP 3 275 376 A1

**Description**

Field

The present invention relates to an ultrasound observation apparatus for observing tissue of an observation target using ultrasound, a method for operating the ultrasound observation apparatus, and a program for operating the ultrasound observation apparatus.

Background

A conventional technique has been known which makes a correction for compensating a reception signal for the frequency-dependent attenuation of living tissue in an ultrasound observation apparatus that observes tissue of an observation target using ultrasound (refer to, for example, Patent Literature 1). In this technique, an ultrasound image is formed using a reception signal obtained by sequentially performing a dynamic correction process in accordance with the depth of a receiving point and a pulse compression process on a reflected wave from a subject.

Citation List

Patent Literature

Patent Literature 1: JP 2010-246640 A

Summary

Technical Problem

However, the technology described in the above-mentioned Patent Literature 1 does not take into account changes in attenuation factor in the depth direction up to a region of interest that is away from the surface of a probe that transmits and receives ultrasound when a correction is made to a reception signal of the region of interest. Hence, an accurate correction can be made only when the attenuation factors of an observation target are uniform, and it is difficult to accurately identify the tissue characteristics of an observation target with non-uniform attenuation factors.

The present invention has been made in view of the foregoing, and an object of the invention is to provide an ultrasound observation apparatus that enables accurate identification of tissue characteristics of an observation target with non-uniform attenuation factors, a method for operating the ultrasound observation apparatus, and a program for operating the ultrasound observation apparatus. Solution to Problem

In order to solve the above described problem and achieve the object, an ultrasound observation apparatus according to the invention generates an ultrasound image based on an ultrasound signal acquired by an ultrasound probe, the ultrasound probe having an ultrasound transducer configured to transmit ultrasound to an observation target and to receive the ultrasound reflected from the observation target. The ultrasound observation apparatus includes: a frequency analysis unit configured to analyze a frequency of the ultrasound signal to calculate a plurality of frequency spectra in accordance with reception depths and receiving directions of the ultrasound signal; a feature calculation unit configured to calculate a feature of each of the plurality of frequency spectra; an attenuation factor setting unit configured to set an attenuation factor of a computation-purpose region, the computation-purpose region being a region that is different from a region of interest in the ultrasound image and is used for computation for correcting the feature; and a feature correction unit configured to perform attenuation correction on the feature using the attenuation factor set by the attenuation factor setting unit, thereby to calculate a corrected feature at a sampling point in the region of interest in the ultrasound image.

In the ultrasound observation apparatus according to the above-described invention, the attenuation factor setting unit is configured to set an attenuation factor of the region of interest in the ultrasound image. The feature correction unit is configured to: calculate a cumulative attenuation factor per unit frequency at the sampling point based on the attenuation factor of the region of interest and the attenuation factor of the computation-purpose region, set by the attenuation factor setting unit; and perform the attenuation correction on the feature using the cumulative attenuation factor, thereby to calculate the corrected feature at the sampling point.

In the ultrasound observation apparatus according to the above-described invention, the computation-purpose region is a region located between a surface of the ultrasound transducer and the region of interest.

In the ultrasound observation apparatus according to the above-described invention, the attenuation factor setting unit is configured to: use each of a plurality of attenuation factor candidate values per unit length and per unit frequency that give different attenuation characteristics when the ultrasound propagates through the observation target, to perform attenuation correction on the feature of each of the frequency spectra for removing an influence of the ultrasound, and thereby to calculate a preliminarily corrected feature of each of the frequency spectra for each of the attenuation factor candidate values; and set an optimum attenuation factor for the observation target from among the plurality of attenuation factor candidate values based on a result of calculating the preliminarily corrected feature.

In the ultrasound observation apparatus according to the above-described invention, the attenuation factor setting unit is configured to divide the computation-purpose region into a plurality of divided regions, and to set the attenuation factor for each of the divided regions.

In the ultrasound observation apparatus according to the above-described invention, the attenuation factor setting unit is configured to divide the region of interest into a plurality of divided regions, and to set the attenuation factor for each of the divided regions.

In the ultrasound observation apparatus according to the above-described invention, the attenuation factor setting unit is configured to set a constant as the attenuation factor of the computation-purpose region.

In the ultrasound observation apparatus according to the above-described invention, the attenuation factor setting unit is configured to: use, as an attenuation factor of the region of interest, each of a plurality of attenuation factor candidate values per unit length and per unit frequency that give different attenuation characteristics when the ultrasound propagates through the observation target, to perform attenuation correction on the feature of each of the frequency spectra for removing an influence of the ultrasound, and thereby to calculate a preliminarily corrected feature of each of the frequency spectra for each of the attenuation factor candidate values; and set an optimum attenuation factor for the observation target from among the plurality of attenuation factor candidate values based on a result of calculating the preliminarily corrected feature.

In the ultrasound observation apparatus according to the above-described invention, the attenuation factor setting unit is configured to set a predetermined fixed value as the attenuation factor of the region of interest.

The ultrasound observation apparatus according to the above-described invention further includes an input unit configured to receive input of information for setting the attenuation factor. The attenuation factor setting unit is configured to set the attenuation factor based on the information received by the input unit.

In the ultrasound observation apparatus according to the above-described invention, in two adjacent divided regions of the plurality of divided regions along a depth direction, one of the two adjacent divided regions located more distant from the ultrasound transducer has a length in the depth direction equal to or more than a length in the depth direction of the other of the two adjacent divided regions located closer to the ultrasound transducer.

In the ultrasound observation apparatus according to the above-described invention, the attenuation factor setting unit is configured to calculate a statistical dispersion of the preliminarily corrected feature for each of the attenuation factor candidate values, and to set an attenuation factor candidate value having a minimal statistical dispersion to be the optimum attenuation factor.

The ultrasound observation apparatus according to the above-described invention further includes a feature image data generation unit configured to generate feature image data for showing information on the corrected feature together with the ultrasound image.

A method for operating an ultrasound observation apparatus according to the invention is a method for operating an ultrasound observation apparatus for generating an ultrasound image based on an ultrasound signal acquired by an ultrasound probe, the ultrasound probe having an ultrasound transducer configured to transmit ultrasound to an observation target and to receive the ultrasound reflected from the observation target. The method includes: a frequency analysis step of analyzing, by a frequency analysis unit, a frequency of the ultrasound signal to calculate a plurality of frequency spectra in accordance with reception depths and receiving directions of the ultrasound signal; a feature calculation step of calculating, by a feature calculation unit, a feature of each of the plurality of frequency spectra; an attenuation factor setting step of setting, by an attenuation factor setting unit, an attenuation factor of a computation-purpose region, the computation-purpose region being a region that is different from a region of interest in the ultrasound image and is used for computation for correcting the feature; and a feature correction step of performing, by a feature correction unit, attenuation correction on the feature using the attenuation factor set in the attenuation factor setting step, thereby to calculate a corrected feature at a sampling point in the region of interest in the ultrasound image.

A program for operating an ultrasound observation apparatus according to the invention is a program for operating an ultrasound observation apparatus for generating an ultrasound image based on an ultrasound signal acquired by an ultrasound probe, the ultrasound probe having an ultrasound transducer configured to transmit ultrasound to an observation target and to receive the ultrasound reflected from the observation target. The program causes the ultrasound observation apparatus to execute: a frequency analysis step of analyzing, by a frequency analysis unit, a frequency of the ultrasound signal to calculate a plurality of frequency spectra in accordance with reception depths and receiving directions of the ultrasound signal; a feature calculation step of calculating, by a feature calculation unit, a feature of each of the plurality of frequency spectra; an attenuation factor setting step of setting, by an attenuation factor setting unit, an attenuation factor of a computation-purpose region, the computation-purpose region being a region that is different from a region of interest in the ultrasound image and is used for computation for correcting the feature; and

a feature correction step of performing, by a feature correction unit, attenuation correction on the feature using the attenuation factor set in the attenuation factor setting step, thereby to calculate a corrected feature at a sampling point in the region of interest in the ultrasound image.

Advantageous Effects of Invention

According to the present invention, an attenuation factor of a computation-purpose region is set. The computation-purpose region is a region that is different from a region of interest in an ultrasound image and that is used for computation for correcting a feature. Attenuation correction is performed on the feature using a result of the setting to calculate a corrected feature at a sampling point in the region of interest in the ultrasound image. Accordingly, even if an attenuation factor of an observation target is non-uniform, the corrected feature can be calculated in view of the non-uniformity. Therefore, according to the present invention, it is possible to accurately identify tissue characteristics of the observation target having the non-uniform attenuation factor.

Brief Description of Drawings

FIG. 1 is a block diagram illustrating a functional configuration of an ultrasound diagnosis system including an ultrasound observation apparatus according to a first embodiment of the present invention.

FIG. 2 is a diagram illustrating the relationship between the reception depth and an amplification factor in an amplification process that is performed by a signal amplification unit of the ultrasound observation apparatus according to the first embodiment of the present invention.

FIG. 3 is a diagram illustrating the relationship between the reception depth and the amplification factor in an amplification correction process that is performed by an amplification correction unit of the ultrasound observation apparatus according to the first embodiment of the present invention.

FIG. 4 is a diagram schematically illustrating data arrangement in one sound ray of an ultrasound signal.

FIG. 5 is a diagram illustrating an example of a frequency spectrum calculated by a frequency analysis unit of the ultrasound observation apparatus according to the first embodiment of the present invention.

FIG. 6 is a diagram schematically illustrating an example of the setting of a region of interest and a computation-purpose region in an ultrasound image according to the first embodiment of the present invention.

FIG. 7 is a diagram illustrating a straight line having, as parameters, preliminarily corrected features corrected by an attenuation factor setting unit of the ultrasound observation apparatus according to the first embodiment of the present invention.

FIG. 8 is a diagram schematically illustrating an example of distributions of preliminarily corrected features that have been corrected for attenuation for the same observation target based respectively on two different attenuation factor candidate values.

FIG. 9 is a flowchart illustrating an overview of a process that is performed by the ultrasound observation apparatus according to the first embodiment of the present invention.

FIG. 10 is a flowchart illustrating an overview of a process that is executed by the frequency analysis unit of the ultrasound observation apparatus according to the first embodiment of the present invention.

FIG. 11 is a diagram illustrating an overview of a process that is performed by the attenuation factor setting unit of the ultrasound observation apparatus according to the first embodiment of the present invention.

FIG. 12 is a diagram schematically illustrating an example of the setting of a region of interest and a computation-purpose region in Modification 1-1 of the first embodiment of the present invention.

FIG. 13 is a diagram illustrating an overview of another method for the setting of an optimum attenuation factor that is performed by the attenuation factor setting unit of the ultrasound observation apparatus according to Modification 1-2 of the first embodiment.

FIG. 14 is a block diagram illustrating a functional configuration of an ultrasound diagnosis system including an ultrasound observation apparatus according to a second embodiment of the present invention.

FIG. 15 is a diagram schematically illustrating an example of the setting of a region of interest and a computation-purpose region in an ultrasound image in the second embodiment of the present invention.

FIG. 16 is a diagram schematically illustrating an example of the setting of a region of interest and a computation-purpose region in an ultrasound image in Modification 2-1 of the second embodiment of the present invention.

FIG. 17 is a diagram schematically illustrating an example of the display of a region of interest on a display device of when an ultrasound observation apparatus according to another embodiment of the present invention is set to a mode that does not use a computation-purpose region.

FIG. 18 is a diagram illustrating another example of the display of a region of interest on the display device of when the ultrasound observation apparatus according to the other embodiment of the present invention is set to a mode that uses the computation-purpose region.

Description of Embodiments

Modes for carrying out the present invention (hereinafter referred to as "embodiment(s)") will be described below with reference to the accompanying drawings.

(First Embodiment)

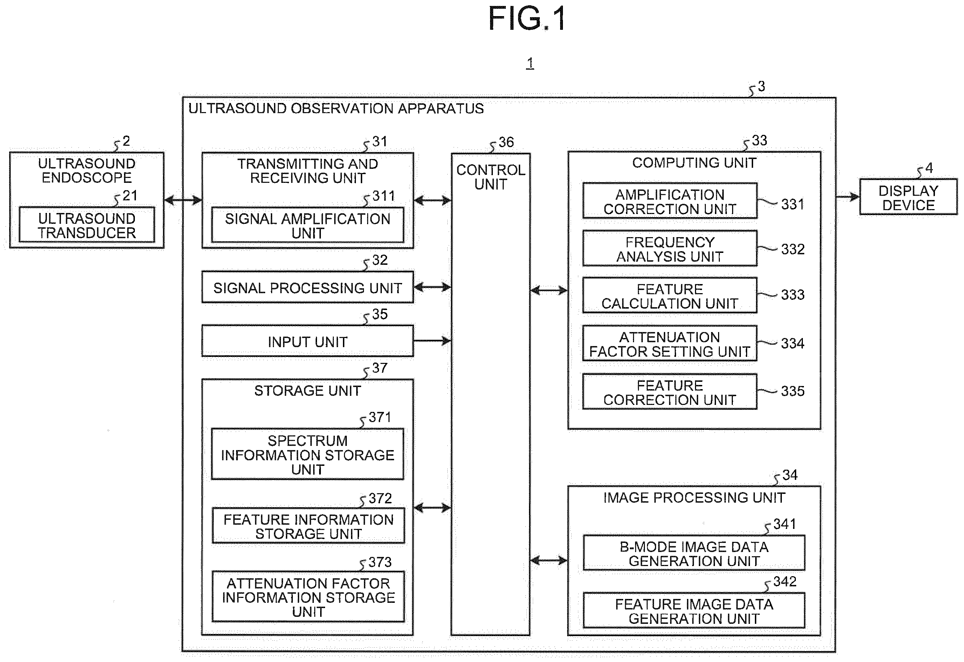

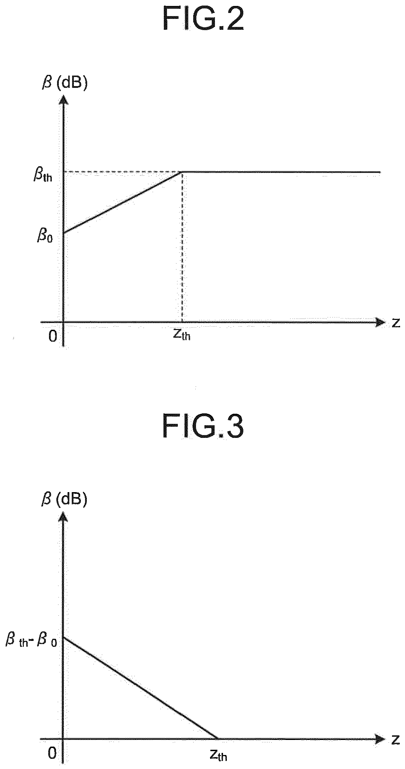

FIG. 1 is a block diagram illustrating a functional configuration of an ultrasound diagnosis system including an ultrasound observation apparatus according to a first embodiment of the present invention. An ultrasound diagnosis system 1 illustrated in FIG. 1 includes an ultrasound endoscope 2 that transmits ultrasound to a subject as an observation target and receives the ultrasound reflected from the subject, an ultrasound observation apparatus 3 that generates an ultrasound image based on the ultrasound signal acquired by the ultrasound endoscope 2, and a display device 4 that displays the ultrasound image generated by the ultrasound observation apparatus 3.
The ultrasound endoscope 2 includes, in its distal end portion, an ultrasound transducer 21 that converts an electric pulsed signal received from the ultrasound observation apparatus 3 into an ultrasound pulse (acoustic pulse) to apply the ultrasound pulse to the subject, and also converts an ultrasound echo reflected from the subject into an electric echo signal expressed as a voltage change to output the echo signal. The ultrasound transducer 21 can be any of a convex transducer, a linear transducer, and a radial transducer. The ultrasound endoscope 2 may be one that scans mechanically with the ultrasound transducer 21, or one that is provided with a plurality of elements in array form as the ultrasound transducer 21 to electronically switch the elements for transmission and reception and delay the transmission and reception by the elements for the purpose of scanning electronically. The ultrasound transducer 21 shall hereinafter be a convex transducer in the first embodiment for explanation purposes.
The ultrasound endoscope 2 generally includes an imaging optical system and an imaging element. The ultrasound endoscope 2 is inserted into the digestive tract (esophagus, stomach, duodenum, or large intestine) or respiratory organ (trachea or bronchus) of the subject to enable the capturing of the digestive tract or respiratory organ, and its surrounding organ (such as pancreas, gallbladder, bile duct, biliary tract, lymph node, mediastinal organ, or blood vessel). Moreover, the ultrasound endoscope 2 includes a light guide that guides illumination light that is applied to the subject upon imaging. The light guide includes a distal end portion that reaches a distal end of an insertion portion into the subject of the ultrasound endoscope 2, and a proximal end portion connected to a light source device that emits the illumination light.
The ultrasound observation apparatus 3 includes: a transmitting and receiving unit 31 that is electrically connected to the ultrasound endoscope 2 to transmit a transmission signal (pulsed signal) including a high voltage pulse to the ultrasound transducer 21 based on a predetermined waveform and transmission timing, and also receive an echo signal being an electric reception signal from the ultrasound transducer 21, and accordingly to generate and output digital radio frequency (RF: Radio Frequency) signal data (hereinafter referred to as RF data); a signal processing unit 32 that generates digital reception data for B-mode based on the RF data received from the transmitting and receiving unit 31; a computing unit 33 that performs a predetermined computation on the RF data received from the transmitting and receiving unit 31; an image processing unit 34 that generates various kinds of image data; an input unit 35 that is realized by user interfaces such as a keyboard, mouse, and touchscreen, and receives input of various kinds of information; a control unit 36 that controls the entire ultrasound diagnosis system 1; and a storage unit 37 that stores various kinds of information necessary for the operation of the ultrasound observation apparatus 3.
The transmitting and receiving unit 31 includes a signal amplification unit 311 that amplifies an echo signal. The signal amplification unit 311 performs Sensitivity Time Control (STC) correction that amplifies an echo signal at a higher amplification factor as the reception depth of the echo signal is increased. FIG. 2 is a diagram illustrating the relationship between the reception depth and the amplification factor in the amplification process that is performed by the signal amplification unit 311. The reception depth z illustrated in FIG. 2 is a quantity calculated based on the time elapsed from the start of reception of ultrasound. As illustrated in FIG. 2, an amplification factor $\beta$ (dB) is increased linearly from $\beta_0$ to $\beta_{th}$ ($> \beta_0$) with increasing reception depth z if the reception depth z is less than a threshold $z_{th}$. Moreover, the amplification factor $\beta$ (dB) takes a constant value $\beta_{th}$ if the reception depth z is equal to or more than the threshold $z_{th}$. The value of the threshold $z_{th}$ is a value that results in the attenuation of most of an ultrasound signal received from an observation target and predominant noise. More generally, the amplification factor $\beta$ is simply required to increase monotonously with increasing reception depth z if the reception depth z is less than the threshold $z_{th}$. The relationship illustrated in FIG. 2 is stored in the storage unit 37 in advance.
After performing processing such as filtering on the echo signal amplified by the signal amplification unit 311, the transmitting and receiving unit 31 performs A/D conversion to generate RF data in the time domain and outputs the RF data to the signal processing unit 32 and the computing unit 33. If the ultrasound endoscope 2 is configured to scan electronically with the ultrasound transducer 21 provided with a plurality of elements in array form, the transmitting and receiving unit 31 has a multi-channel circuit for beam synthesis corresponding to the plurality of elements.

A frequency band of the pulsed signal transmitted by the transmitting and receiving unit 31 is desirably a wide band that covers most of a linear response frequency band of electroacoustic conversion from a pulsed signal to an ultrasound pulse in the ultrasound transducer 21. Moreover, various processing frequency bands of the echo signal in the signal amplification unit 311 is desirably a wide band that covers most of a linear response frequency band of electroacoustic conversion from an ultrasound echo to an echo signal by the ultrasound transducer 21. Consequently, it becomes possible to accurately make an approximation in executing a frequency spectrum approximation process, which will be described below.

The transmitting and receiving unit 31 also has a function of transmitting various control signals output by the control unit 36 to the ultrasound endoscope 2 and also receiving various kinds of information including an ID for identification from the ultrasound endoscope 2 to transmit the information to the control unit 36.

The signal processing unit 32 performs known processing such as band-pass filtering, envelope detection, and logarithmic conversion on the RF data, and generates digital reception data for B-mode. In the logarithmic conversion, a decibel value representation is used taking the common logarithm of a quantity obtained by dividing the RF data by a reference voltage. The signal processing unit 32 outputs the generated reception data for B-mode to the image processing unit 34. The signal processing unit 32 is realized by a general-purpose processor such as a CPU (Central Processing Unit), or a dedicated integrated circuit that executes a specific function, such as an Application Specific Integrated Circuit (ASIC) or Field Programmable Gate Array (FPGA), or the like.

The computing unit 33 includes: an amplification correction unit 331 that performs amplification correction on the RF data output by the transmitting and receiving unit 31 to make the amplification factor constant irrespective of the reception depth; a frequency analysis unit 332 that performs the fast Fourier transform (FFT) on the RF data on which the amplification correction has been performed for a frequency analysis and, accordingly, to calculate a plurality of frequency spectra in accordance with the reception depths and receiving directions of an ultrasound signal; a feature calculation unit 333 that calculates features of each frequency spectrum; an attenuation factor setting unit 334 that sets attenuation factors for a region of interest and a computation-purpose region being a region that is different from the region of interest, the region being used for computation for correcting the features, in an ultrasound image; and a feature correction unit 335 that uses the attenuation factors that have been set respectively for the region of interest and the computation-purpose region by the attenuation factor setting unit 334 to calculate corrected features at a sampling point in the region of interest. The computing unit 33 is realized by using a general-purpose processor such as a CPU, a dedicated integrated circuit such as an ASIC or FPGA, or the like.

FIG. 3 is a diagram illustrating the relationship between the reception depth and the amplification factor in the amplification correction process that is performed by the amplification correction unit 331. As illustrated in FIG. 3, the amplification factor $\beta$ (dB) in the amplification correction process that is performed by the amplification correction unit 331 takes a maximum value $\beta_{th} - \beta_0$ when the reception depth z is zero, reduces linearly until the reception depth z reaches from zero to the threshold $z_{th}$, and is zero when the reception depth z is equal to or more than the threshold $z_{th}$. The relationship illustrated in FIG. 3 is stored in advance in the storage unit 37. The amplification correction unit 331 performs amplification correction on the digital RF data based on the relationship illustrated in FIG. 3. Accordingly, it is possible to cancel the influence of the STC correction in the signal amplification unit 311 and output a signal with a constant amplification factor $\beta_{th}$. The relationship between the reception depth z and the amplification factor $\beta$ that is performed by the amplification correction unit 331 is naturally different depending on the relationship between the reception depth and the amplification factor in the signal amplification unit 311.

The reason why such amplification correction is performed will be hereinafter described. The STC correction is a correction process that amplifies the amplitude of an analog signal waveform uniformly over the entire frequency band and with the amplification factor that increases monotonously with respect to the depth, and accordingly removes the influence of attenuation from the amplitude of the analog signal waveform. Hence, if a B-mode image is generated which displays the amplitude of an echo signal after conversion into luminance, and if uniform tissue is scanned, the luminance value becomes constant irrespective of the depth due to the performance of the STC correction. In other words, it is possible to obtain an effect that removes the influence of attenuation from the luminance value of the B-mode image.

On the other hand, if the calculation and analysis result of a frequency spectrum of ultrasound is used as in the first embodiment, there is a problem that the influence of attenuation associated with the propagation of ultrasound cannot always be removed accurately even in the STC correction. This is because the amount of attenuation is generally different depending on the frequency (refer to equation (1) described below), but the amplification factor of the STC correction changes according only to the distance and is not frequency dependent.

In order to solve the above-mentioned problem, it is conceivable that a reception signal on which the STC correction has been performed is output when a B-mode image is generated, while, at the time of generating an image based on a frequency spectrum, a new transmission different from the transmission for generating the B-mode image is performed to output a reception signal on which the STC correction has not been performed. However, in this case, there is a problem that the frame rate of image data generated based on the reception signal is reduced.

Hence, in the first embodiment, the amplification correction unit 331 corrects the amplification factor for a signal on which the STC correction has been performed for a B-mode image in order to remove the influence of the STC correction while maintaining the frame rate of image data generated.

The frequency analysis unit 332 samples RF data (line data) of each sound ray corrected for amplification by the amplification correction unit 331 at predetermined time intervals, and generates sample data. The frequency analysis unit 332 performs the FFT process on sample data groups to calculate a frequency spectrum at a plurality of points (data positions) on the RF data.

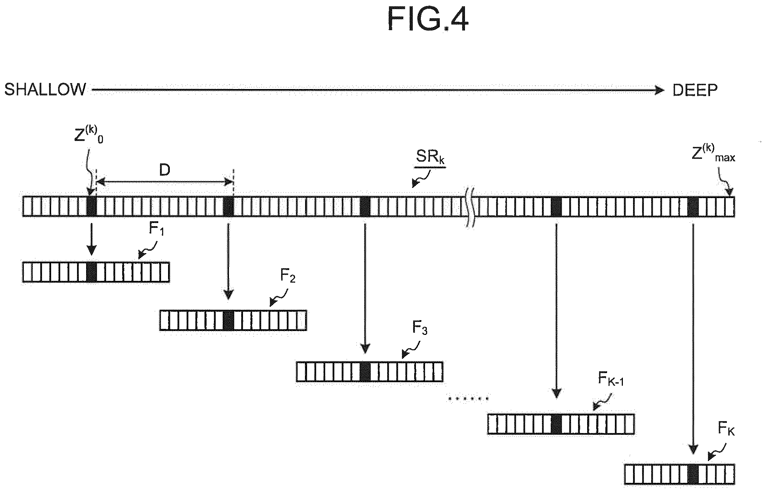

FIG. 4 is a diagram schematically illustrating data arrangement in one sound ray of an ultrasound signal. In a sound ray $SR_k$ illustrated in FIG. 4, a white or black rectangle indicates data at one sample point. Moreover, in the sound ray $SR_k$, data located at a further right position is sample data from a deeper point when measurements are taken along the sound ray $SR_k$ from the ultrasound transducer 21 (refer to an arrow in FIG. 4). The sound ray $SR_k$ is discretized at intervals of time corresponding to a sampling frequency (for example, 50 MHz) in A/D conversion performed by the transmitting and receiving unit 31. FIG. 4 illustrates a case where the position of the eighth data of the sound ray $SR_k$ of number k is set as an initial value $Z^{(k)}_0$ in the direction of the reception depth z. However, the position of the initial value can be set freely. The result of the calculation made by the frequency analysis unit 332 is obtained in a complex number and stored in the storage unit 37.

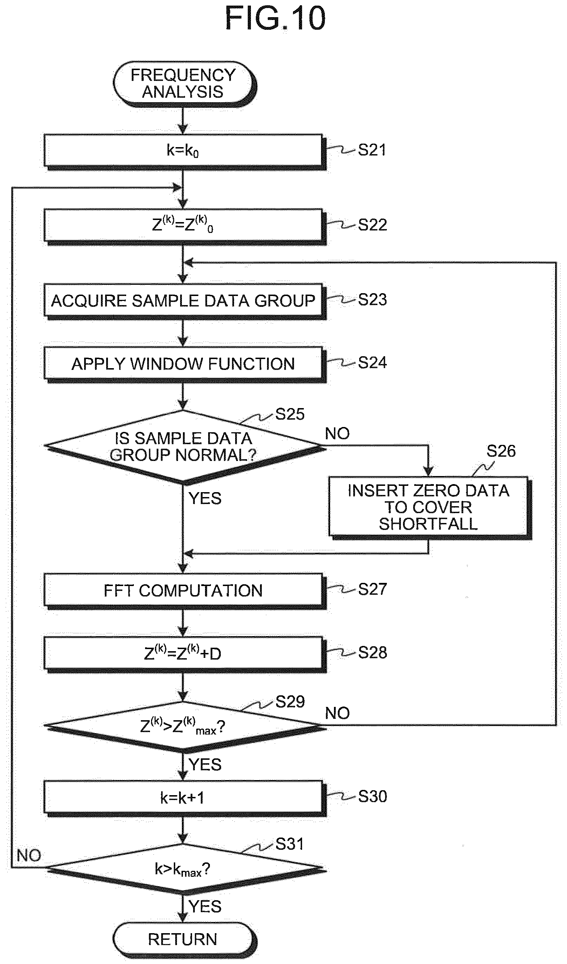

A data group $F_j$ (j = 1, 2, ..., K) illustrated in FIG. 4 is a sample data group targeted for the FFT process. The sample data group is generally required to have the number of pieces of data being a power of 2 for the FFT process. In this sense, the sample data group $F_j$ (j = 1, 2, ..., K-1) includes 16 (= $2^4$) pieces of data and is a normal data group, while a sample data group $F_k$ includes 12 pieces of data and therefore is an abnormal data group. When the FFT process is performed on the abnormal data group, a process is performed in which zero data covering the shortfall is inserted to generate a normal sample data group. In this regard, a detailed description is given when the process of the frequency analysis unit 332 is described (refer to FIG. 10).

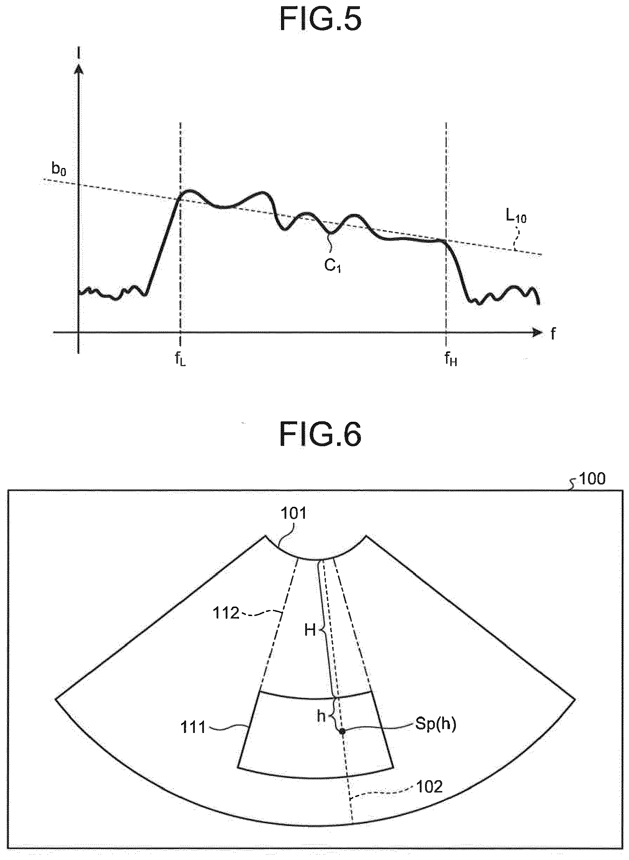

FIG. 5 is a diagram illustrating an example of the frequency spectrum calculated by the frequency analysis unit 332. "Frequency spectrum" here indicates the "distribution of frequencies at an intensity at some reception depth z" obtained by performing the FFT process on a sample data group. Moreover, "intensity" here indicates any of, for example, parameters such as the voltage of an echo signal, the power of the echo signal, the sound pressure of the ultrasound echo, and the sound energy of the ultrasound echo, the amplitudes and time integrated values of these parameters, and their combinations.

In FIG. 5, the horizontal axis represents a frequency f. Moreover, in FIG. 5, the vertical axis represents the common logarithm (expressed in decibels) of a quantity obtained by dividing an intensity $I_0$ by a reference intensity $I_c$ (constant), $I = 10 \log_{10} (I_0 / I_c)$. In FIG. 5, the reception depth z is constant. A straight line $L_{10}$ illustrated in FIG. 5 is described below. In the first embodiment, a curve and a straight line each include a set of discrete points.

In a frequency spectrum $C_1$ illustrated in FIG. 5, a lower-limit frequency $f_L$ and an upper-limit frequency $f_H$ of a frequency band, which is used for a subsequent computation, are parameters that are determined based on the frequency band of the ultrasound transducer 21, the frequency band of a pulsed signal transmitted by the transmitting and receiving unit 31, and the like. For example, $f_L$ = 3 MHz, and $f_H$ = 10 MHz. In FIG. 5, the frequency band determined by the lower-limit frequency $f_L$ and the upper-limit frequency $f_H$ is hereinafter referred to as "frequency band U."

If the observation target is living tissue, the frequency spectrum generally indicates a different tendency depending on the characteristics of the living tissue that is scanned with ultrasound. This is because the frequency spectrum has a correlation to the size, number density, acoustic impedance, and the like of a scatterer that scatters ultrasound. The "characteristics of the living tissue" herein indicates, for example, a malignant tumor (cancer), a benign tumor, an endocrine tumor, a mucinous tumor, normal tissue, a cyst, and a vas.

The feature calculation unit 333 performs a regression analysis on a frequency spectrum in a predetermined frequency band and approximates the frequency spectrum by a linear expression, thereby calculating features that characterize the linear expression approximated. For example, in a case of the frequency spectrum $C_1$ illustrated in FIG. 5, the feature calculation unit 333 performs a regression analysis in the frequency band U to obtain the approximate straight line $L_{10}$. Suppose that the approximate straight line $L_{10}$ is expressed below in a linear expression of the frequency f, $I = a_0 f + b_0$. The feature calculation unit 333 calculates, as the features corresponding to the straight line $L_{10}$, a slope $a_0$, an intercept $b_0$, and a midband fit $c_0 = a_0 f_M + b_0$ that is a value of the intensity I at a center frequency in the frequency band U, $f_M = (f_s + f_e)/2$. The feature calculation unit 333 may approximate the frequency spectrum by a quadratic or higher order polynomial using a regression analysis.

Among the three features before correction, the slope $a_0$ is considered to have a correlation with the size of the scatterer of ultrasound, and generally have a smaller value as the scatterer is increased in size. Moreover, the intercept $b_0$ has a correlation with scatterer size, difference in acoustic impedance, scatterer number density (concentration), and the like. Specifically, the intercept $b_0$ is considered to have a larger value as the scatterer is increased in size, have a larger value as the difference in acoustic impedance is increased, and have a larger value as the number density of the scatterer is increased. Also, the midband fit $c_0$ is an indirect parameter that is derived from the slope $a_0$ and the intercept $b_0$, and gives the intensity of a spectrum at the center of the effective frequency band. Hence, the midband fit $c_0$ is considered to have some correlation with the luminance of a B-mode image in addition to the scatterer size, the difference in acoustic impedance, and the scatterer number density.

The attenuation factor setting unit 334 performs attenuation correction on the features of each frequency spectrum for removing an influence of the ultrasound in the region of interest and the computation-purpose region that are set in the ultrasound image, using each of a plurality of attenuation factor candidate values per unit length and per unit frequency that give different attenuation characteristics when the ultrasound propagates through the observation target, to calculate preliminarily corrected features of each frequency spectrum for each attenuation factor candidate value, and sets an optimum attenuation factor for the observation target among the plurality of attenuation factor candidate values based on the calculation result.

FIG. 6 is a diagram schematically illustrating an example of the setting of a region of interest and a computation-purpose region in an ultrasound image. FIG. 6 is described omitting the ultrasound image itself. The point of the description where the ultrasound image itself is omitted is also similar in drawings of an ultrasound image, which are described below. Moreover, FIG. 6 illustrates only one sound ray (a sound ray 102) by example. It is needless to say, however, that a plurality of sound rays is set at predetermined intervals along a scan angle direction.

A region of interest 111 and a computation-purpose region 112 located between a surface position 101 of the ultrasound transducer 21 and the region of interest 111 are set in an ultrasound image 100 illustrated in FIG. 6. The region of interest 111 is a region surrounded by a total of four borders of two borders extending in a straight line from the surface position 101 along a depth direction, and two borders having an arc shape along the scan angle direction. The computation-purpose region 112 touches the surface position 101 and the region of interest 111. Moreover, among borders of the computation-purpose region 112, two borders extending in a straight line from the surface position 101 along the depth direction are included in the same straight lines of the two borders of the region of interest 111 extending in a straight line from the surface position 101 along the depth direction. When the ultrasound image is displayed on the display device 4, the borders (solid lines of FIG. 6) of the region of interest 111 are displayed while the computation-purpose region 112 is not displayed. However, as illustrated in FIG. 6, it is also possible to display the computation-purpose region 112 on the display device 4 in a mode that is distinguishable from the region of interest 111.

In FIG. 6, the region of interest 111 touches the computation-purpose region 112. However, it is also possible to set them to intersect partly. For example, the two regions may be set such that a portion of the region of interest 111 that is small in depth and a portion of the computation-purpose region 112 that is large in depth have a band-shaped overlap along the scan angle direction.

The amount of attenuation A (f, z) of ultrasound is attenuation that occurs while ultrasound travels between the reception depth 0 and the reception depth z, and is defined as an intensity change (a difference expressed in decibels) before and after the round trip. The amount of attenuation A (f, z) is empirically known to be proportional to the frequency in uniform tissue, and is expressed by the following equation (1):

$$A(f, z) = 2\alpha z f \qquad \dots (1).$$

Here, the proportionality constant $\alpha$ is a quantity called the attenuation factor, and gives the amount of attenuation of ultrasound per unit length and per unit frequency. Moreover, z is the reception depth of ultrasound, and f is the frequency. If the observation target is a living body, a specific value of the attenuation factor $\alpha$ is determined in accordance with the region of the living body. The unit of the attenuation factor $\alpha$ is, for example, dB/cm/MHz.

The attenuation factor setting unit 334 sets an optimum attenuation factor from a plurality of attenuation factor candidate values. At this point in time, the attenuation factor setting unit 334 performs attenuation correction on the features (the slope $a_0$, the intercept $b_0$, and the midband fit $c_0$) calculated by the feature calculation unit 333 in accordance with equations (2) to (4) illustrated below, using the attenuation factor candidate value $\alpha$, to calculate preliminarily corrected features a, b, and c.

$$a = a_0 + 2\alpha z \qquad \dots (2)$$

$$b = b_0 \qquad \dots (3)$$

$$c = c_0 + A(f_M, z) = c_0 + 2\alpha z f_M \ (= af_M + b) \qquad \dots \ (4)$$

As is clear from equations (2) and (4), the attenuation factor setting unit 334 makes corrections in which the amount of correction is increased with increasing reception depth z of ultrasound. Moreover, according to equation (3), the correction related to the intercept is the identity transformation. This is because the intercept is a frequency component corresponding to a frequency of zero (Hz) and is not influenced by attenuation.

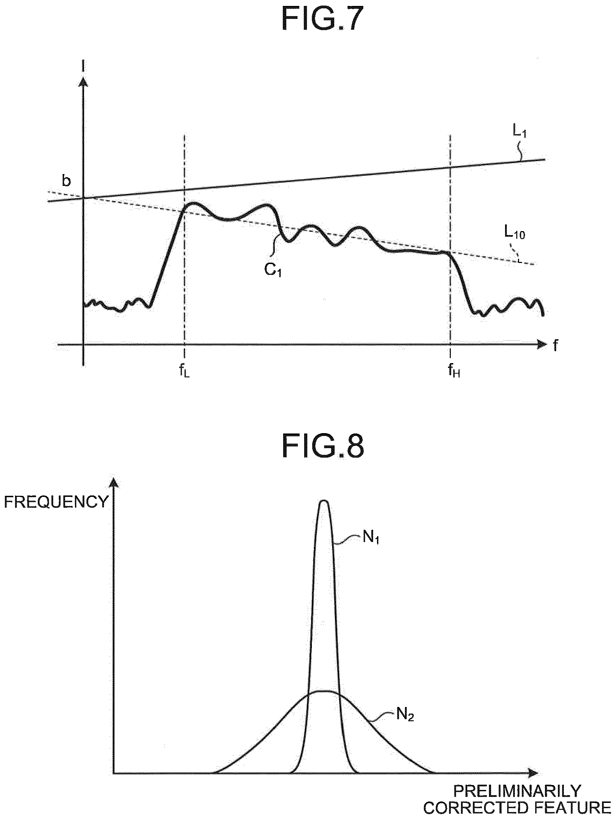

FIG. 7 is a diagram illustrating a straight line having, as parameters, the preliminarily corrected features a, b, and c corrected as the parameters by the attenuation factor setting unit 334. An equation of a straight line $L_1$ is expressed as

$$I = af + b = (a_0 + 2\alpha z)f + b_0 \ \dots \ (5).$$

As is clear from equation (5), the straight line $L_1$ is larger in slope ($a > a_0$) and the same in intercept ($b = b_0$) compared to the straight line $L_{10}$ before attenuation correction.

The attenuation factor setting unit 334 sets an attenuation factor candidate value having minimal statistical dispersion of the preliminarily corrected feature calculated for each attenuation factor candidate value, as the optimum attenuation factor, in each of the region of interest and the computation-purpose region. In the first embodiment, the variance is applied as a quantity indicating statistical dispersion. In this case, the attenuation factor setting unit 334 sets an attenuation factor candidate value having a minimum variance as the optimum attenuation factor. Two of the above-mentioned three preliminarily corrected features a, b, and c are independent. In addition, the preliminarily corrected feature b is not dependent on the attenuation factor. Therefore, if the optimum attenuation factor is set for the preliminarily corrected features a and c, the attenuation factor setting unit 334 is simply required to calculate the variance of one of the preliminarily corrected features a and c.

However, it is preferable that the preliminarily corrected feature that is used when the attenuation factor setting unit 334 sets the optimum attenuation factor be the same kind as the corrected feature that is used when a feature image data generation unit 342 generates feature image data. In other words, it is preferable to apply the variance of the preliminarily corrected feature a if the feature image data generation unit 342 generates feature image data using the slope as the corrected feature while it is preferable to apply the variance of the preliminarily corrected feature c if the feature image data generation unit 342 generates feature image data using the midband fit as the corrected feature. This is because equation (1) that gives the amount of attenuation A (f, z) is simply ideal, but the following equation (6) is more appropriate in practice.

$$A(f, z) = 2\alpha z f + 2\alpha_1 z \qquad \dots \ (6)$$

$\alpha_1$ of the second term on the right-hand side of equation (6) is a coefficient indicating the magnitude of a change in signal intensity in proportion to the reception depth z of ultrasound, and is a coefficient indicating a change in signal intensity that occurs due to non-uniformity of tissue of the observation target, a change in the number of channels upon beam synthesis, and the like. Because of the second term on the right-hand side of equation (6), when the midband fit is used as the corrected feature to generate feature image data, it is possible to correct attenuation more accurately by the setting of the optimum attenuation factor using the variance of the preliminarily corrected feature c (refer to equation (4)). On the other hand, if the slope being a coefficient that is proportional to the frequency f is used as the corrected feature to generate feature image data, it is possible to remove the influence of the second term on the right-hand side of equation (6) and correct attenuation more accurately by the setting of the optimum attenuation factor using the variance of the preliminarily corrected feature a.

The reason why the optimum attenuation factor can be set based on statistical dispersion will be hereinafter described. It is considered that, if the optimum attenuation factor is applied to an observation target, a feature converges to a value specific to the observation target irrespective of the distance between the observation target and the ultrasound transducer 21, and statistical dispersion is reduced. On the other hand, it is considered that, if an attenuation factor candidate value that is not fit for the observation target is set as the optimum attenuation factor, the attenuation correction is excessive or insufficient. Accordingly, the feature varies according to the distance to the ultrasound transducer 21, and the statistical dispersion of the feature is increased. Therefore, it can be said that an attenuation factor candidate value having minimal statistical dispersion is the optimum attenuation factor for the observation target.

FIG. 8 is a diagram schematically illustrating an example of distributions of preliminarily corrected features that have been corrected for attenuation for the same observation target based respectively on two different attenuation factor candidate values. In FIG. 8, the horizontal axis indicates the preliminarily corrected feature, and the vertical axis indicates the frequency. Two distribution curves $N_1$ and $N_2$ illustrated in FIG. 8 have the same sum of frequencies. In the case illustrated in FIG. 8, the distribution curve $N_1$ has less statistical dispersion (less variance) of the feature than the distribution curve $N_2$, and has a crest that forms a steep shape. Therefore, when setting the optimum attenuation factor from the two attenuation factor candidate values corresponding to the two distribution curves $N_1$ and $N_2$, the attenuation factor setting unit 334 sets the attenuation factor candidate value corresponding to the distribution curve $N_1$ as the optimum attenuation factor.

The feature correction unit 335 calculates a cumulative attenuation factor per unit frequency at a sampling point (hereinafter simply referred to as the cumulative attenuation factor) using the optimum attenuation factors that have been set respectively for the region of interest and the computation-purpose region, and performs attenuation correction on the features using the cumulative attenuation factor. Specifically, a cumulative attenuation factor $y(h)$, for example, at a sampling point Sp(h) whose distance from the border on a side close to the ultrasound transducer 21 is h in the region of interest 111, the sampling point Sp(h) being on the sound ray 102 illustrated in FIG. 6, is expressed as:

$$\gamma(h) = 2H \cdot \alpha_c + 2h \cdot \alpha_{ROI} \quad \dots \quad (7).$$

Here, the first term on the right-hand side is the product of the round-trip distance 2H of ultrasound in the depth direction from the surface position 101 of the ultrasound transducer 21 and the region of interest 111 (the round-trip distance of ultrasound in the computation-purpose region 112) and the optimum attenuation factor $a_c$ in the computation-purpose region 112. Moreover, the second term on the right-hand side is the product of the round-trip distance 2h of ultrasound in the depth direction in the region of interest 111 and the optimum attenuation factor $\alpha_{ROI}$ in the region of interest 111. In this manner, the feature correction unit 335 accumulates the attenuation factors from the surface of the ultrasound transducer 21 to calculate the cumulative attenuation factor. Assuming that the unit of the optimum attenuation factor is dB/cm/MHz, the unit of the cumulative attenuation factor is dB/MHz.

The feature correction unit 335 corrects the features at a sampling point using the cumulative attenuation factor. Specifically, the feature correction unit 335 performs attenuation correction on the features at, for example, the sampling point Sp(h) illustrated in FIG. 6, using the cumulative attenuation factor y(h) at the sampling point Sp(h), as in the following equations (8) to (10) to calculate corrected features a(h), b(h), and c(h).

$$a(h) = a_0 + 2\gamma(h) \quad \dots \quad (8)$$

$$b(h) = b_0 \quad \dots \quad (9)$$

$$c(h) = c_0 + 2f_M\gamma(h) \quad \dots \quad (10)$$

The image processing unit 34 includes a B-mode image data generation unit 341 that generates B-mode image data being an ultrasound image that displays the amplitude of an echo signal after conversion into luminance, and a feature image data generation unit 342 that generates feature image data that shows information on the features calculated by the feature calculation unit 333.

The B-mode image data generation unit 341 performs signal processing on the reception data for B-mode received from the signal processing unit 32, using known technologies such as gain processing and contrast processing, and also, for example, reduces data in accordance with the data step width determined according to the image display range of the display device 4. Accordingly, the B-mode image data generation unit 341 generates B-mode image data. A B-mode image is a grayscale image where the values of R (red), G (green), and B (blue), which are variables when the RGB color system is adopted as the color space, are made equal.

The B-mode image data generation unit 341 performs coordinate conversion on the reception data for B-mode for rearrangement so as to spatially represent scanning ranges correctly, and then performs interpolation between the reception data for B-mode. Accordingly, the B-mode image data generation unit 341 bridges gaps between the reception data for B-mode, and generates B-mode image data. The B-mode image data generation unit 341 outputs the generated B-mode image data to the feature image data generation unit 342.

The feature image data generation unit 342 superimposes visual information related to the feature(s) calculated by the feature calculation unit 333 on each pixel of an image of the B-mode image data, and accordingly generates feature image data. The feature image data generation unit 342 assigns visual information corresponding to the feature(s) of a frequency spectrum calculated from, for example, one amplification data group $F_j$ (j = 1, 2, ..., K) illustrated in FIG.

4 to a pixel region corresponding to the data amount of the amplification data group $F_j$. The feature image data generation unit 342 associates hue as the visual information with any of, for example, the above-mentioned slope, intercept, and midband fit to generate feature image data. The feature image data generation unit 342 may associate hue with one of two features selected from the slope, intercept, and midband fit, and associate contrast with the other, to generate feature image data. The visual information on the feature(s) include variables of a color space forming a predetermined color system such as saturation, luminance value, R (red), G (green), and B (blue), in addition to hue and contrast (brightness).

The control unit 36 is realized using a general-purpose processor such as a CPU having a computation and control function, a dedicated integrated circuit such as an ASIC or FPGA, or the like. If the control unit 36 is realized by a general-purpose processor or FPGA, the control unit 36 reads various programs and various kinds of data, which are stored in the storage unit 37, from the storage unit 37, executes various computation processes related to a method for operating the ultrasound observation apparatus 3, and accordingly integrally controls the ultrasound observation apparatus 3. If the control unit 36 is configured using an ASIC, the control unit 36 may execute various processes alone or may execute various processes using various kinds of data and the like stored in the storage unit 37. The control unit 36 may share a common general-purpose processor, a dedicated integrated circuit, or the like with the signal processing unit 32 and the computing unit 33.

The storage unit 37 includes a spectrum information storage unit 371 that stores information on the frequency spectra calculated by the frequency analysis unit 332 together with the reception depths and the receiving directions, a feature information storage unit 372 that stores information on the features calculated by the feature calculation unit 333 and the corrected features corrected by the feature correction unit 335, and an attenuation factor information storage unit 373 that stores information on the optimum attenuation factors that are set respectively for the region of interest and the computation-purpose region by the attenuation factor setting unit 334 and a cumulative attenuation factor at each sampling point in the region of interest, the cumulative attenuation factor being calculated by the feature correction unit 335.

In addition to the above information, the storage unit 37 stores, for example, information necessary for the amplification process (the relationship between the amplification factor and the reception depth illustrated in FIG. 2), information necessary for the amplification correction process (the relationship between the attenuation factor and the reception depth illustrated in FIG. 3), information necessary for the attenuation correction process (refer to equation (1)), and information on a window function (such as Hamming, Hanning, or Blackman) necessary for the frequency analysis process.

Moreover, the storage unit 37 stores various programs including an operation program for executing the method for operating the ultrasound observation apparatus 3. The operation program can also be recorded in computer-readable recording media such as hard disks, flash memories, CD-ROMs, DVD-ROMs, and flexible disks to be put into general circulation. The above-mentioned various programs can also be acquired by being downloaded via communication networks. The communication networks here are realized by, for example, an existing public network, Local Area Network (LAN), and Wide Area Network (WAN) irrespective of wired or wireless.

The storage unit 37 having the above configuration is realized by, for example, a Read Only Memory (ROM) in which various programs and the like are preinstalled, and a Random Access Memory (RAM) in which computational parameters, data, and the like of various processes are stored.

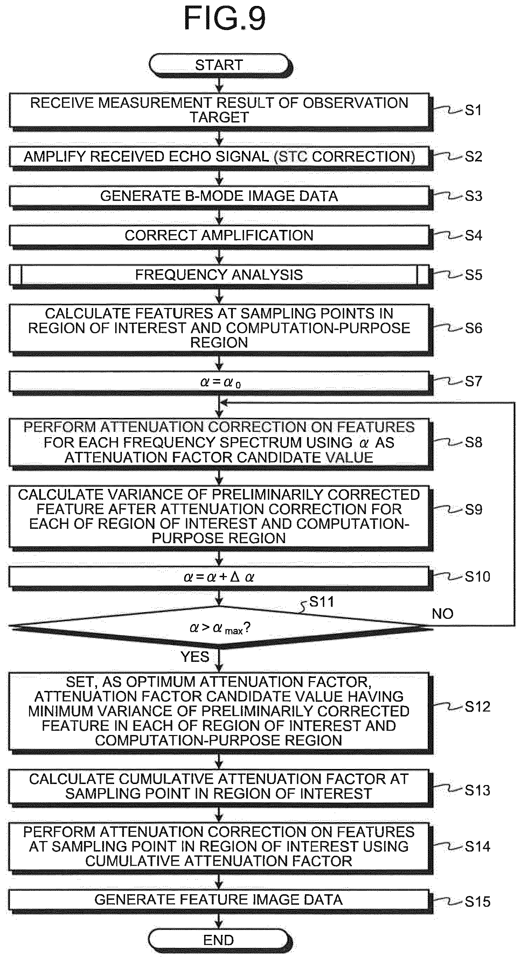

FIG. 9 is a flowchart illustrating an overview of a process that is performed by the ultrasound observation apparatus 3 having the above configuration. Specifically, FIG. 9 is a flowchart illustrating an overview of a process after the ultrasound observation apparatus 3 receives an echo signal from the ultrasound endoscope 2. The process that is performed by the ultrasound observation apparatus 3 is described hereinafter with reference to FIG. 9. First, the ultrasound observation apparatus 3 receives, from the ultrasound endoscope 2, an echo signal as a measurement result of the observation target by the ultrasound transducer 21 (Step S1).

The signal amplification unit 311, which has received the echo signal from the ultrasound transducer 21, amplifies the echo signal (Step S2). The signal amplification unit 311 performs an amplification (the STC correction) on the echo signal based on, for example, the relationship between the amplification factor and the reception depth illustrated in FIG. 2.

Next, the B-mode image data generation unit 341 generates B-mode image data using the echo signal amplified by the signal amplification unit 311, and outputs the B-mode image data to the display device 4 (Step S3). The display device 4, which has received the B-mode image data, displays a B-mode image corresponding to the B-mode image data.

The amplification correction unit 331 performs amplification correction on the RF data output from the transmitting and receiving unit 31 such that the amplification factor is constant irrespective of the reception depth (Step S4). The amplification correction unit 331 performs amplification correction based on, for example, the relationship between the amplification factor and the reception depth illustrated in FIG. 3.

The frequency analysis unit 332 then performs a frequency analysis by the FFT on the RF data of each sound ray after amplification correction to calculate frequency spectra for all sample data groups, and stores them in the spectrum information storage unit 371 (Step S5). FIG. 10 is a flowchart illustrating an overview of the process that is performed by the frequency analysis unit 332 in Step S5. The details of the frequency analysis process are described hereinafter with reference to the flowchart illustrated in FIG. 10.

First, the frequency analysis unit 332 sets a counter k that identifies a sound ray targeted for analysis to $k_0$ (Step S21).

Next, the frequency analysis unit 332 sets an initial value $Z^{(k)}_0$ of a data position (corresponding to the reception depth) $Z^{(k)}$ representing a series of data groups (sample data groups) generated for FFT computation (Step S22). For example, FIG. 4 illustrates the case where the position of the eighth data of the sound ray $SR_k$ is set as an initial value $Z^{(k)}_0$ as described above.

Next, the frequency analysis unit 332 acquires the sample data groups (Step S23), and applies a window function stored in the storage unit 37 to the acquired sample data groups (Step S24). The window function is applied to the sample data groups in this manner and accordingly, it is possible to avoid the sample data groups from becoming discontinuous at their boundaries and prevent the occurrence of an artifact.

Next, the frequency analysis unit 332 determines whether or not the sample data group at the data position $Z^{(k)}$ is a normal data group (Step S25). As described with reference to FIG. 4, the sample data group needs to include the number of pieces of data being a power of 2. The number of pieces of data of a normal sample data group shall be $2^n$ (n is a positive integer) below. In the first embodiment, the data position $Z^{(k)}$ is set to be located as close to the center of a sample data group to which $Z^{(k)}$ belongs as possible. Specifically, since the number of pieces of data of a sample data group is $2^n$, $Z^{(k)}$ is set at the $2^n/2$ (= $2^{n-1}$)-th position close to the center of the sample data group. In this case, that the sample data group is normal indicates that there are $2^{n-1} - 1$ (= N) pieces of data on the shallower side than the data position $Z^{(k)}$, and there are $2^{n-1}$ (= M) pieces of data on the deeper side than the data position $Z^{(k)}$. In the case of FIG. 4, the sample data group $F_j$ (j = 1, 2, ..., K - 1) is normal. FIG. 4 illustrates a case where n = 4 (N = 7, M = 8).

If the sample data group at the data position $Z^{(k)}$ is normal as the result of the determination in Step S25 (Step S25: Yes), the frequency analysis unit 332 proceeds to Step S27 described below.

If the sample data group at the data position $Z^{(k)}$ is not normal as the result of the determination in Step S25 (Step S25: No), the frequency analysis unit 332 generates a normal sample data group by inserting zero data for covering the shortfall (Step S26). The sample data group determined to be not normal in Step S25 (for example, the sample data group $F_K$ of FIG. 4) is applied a window function before being added zero data. Hence, even if zero data is inserted into the sample data group, the discontinuity of data does not occur. After Step S26, the frequency analysis unit 332 proceeds to Step S27 described below.

In Step S27, the frequency analysis unit 332 performs the FFT computation using a sample data group to obtain a frequency spectrum being the amplitude-frequency distribution (Step S27).

Next, the frequency analysis unit 332 changes the data position $Z^{(k)}$ by a step width D (Step S28). The step width D is stored in the storage unit 37 in advance. FIG. 4 illustrates a case where D = 15. The step width D is desired to agree with the data step width that is used when the B-mode image data generation unit 341 generates B-mode image data. However, if the amount of computation in the frequency analysis unit 332 is desired to be reduced, a larger value than the data step width may be set as the step width D.

The frequency analysis unit 332 then determines whether or not the data position $Z^{(k)}$ is larger than a maximum value $Z^{(k)}_{max}$ of the sound ray $SR_k$ (Step S29). If the data position $Z^{(k)}$ is larger than the maximum value $Z^{(k)}_{max}$ (Step S29: Yes), the frequency analysis unit 332 increments the counter k by one (Step S30). This indicates a shift of the processing to the adjacent sound ray. On the other hand, if the data position $Z^{(k)}$ is equal to or less than the maximum value $Z^{(k)}_{max}$ (Step S29: No), the frequency analysis unit 332 returns to Step S23.

After Step S30, the frequency analysis unit 332 determines whether or not the counter k is larger than the maximum value $k_{max}$ (Step S31). If the counter k is larger than $k_{max}$ (Step S31: Yes), the frequency analysis unit 332 ends a series of the frequency analysis process steps. On the other hand, if the counter k is equal to or less than $k_{max}$ (Step S31: No), the frequency analysis unit 332 returns to Step S22. The maximum value $k_{max}$ is a value that a user such as an operator has input through the input unit 35, or a value that is preset in the storage unit 37.

In this manner, the frequency analysis unit 332 performs FFT computation multiple times on each of ($k_{max} - k_0$ + 1) sound rays within an analysis target region. The frequency spectra obtained as the result of the FFT computation, together with the reception depths and the receiving directions, are stored in the spectrum information storage unit 371.

In the above explanation, the frequency analysis unit 332 performs the frequency analysis process on all the regions that have received the ultrasound signal. However, the input unit 35 may receive setting and input of a partial region divided by a specific depth width and sound ray width, and the frequency analysis process may be performed on only the set partial region.

Subsequent to the frequency analysis process of Step S5 described above, the feature calculation unit 333 calculates features of a frequency spectrum at a sampling point included in each of the region of interest and the computation-purpose region (Step S6). Specifically, the feature calculation unit 333 performs a regression analysis on a frequency spectrum in a predetermined frequency band to approximate the frequency spectrum by a linear expression $I = a_0 f + b_0$, and calculates the slope $a_0$, the intercept $b_0$, and the midband fit $c_0$ as the features. For example, the straight line $L_{10}$ illustrated in FIG. 5 is a regression line obtained by the feature calculation unit 333 approximating the frequency

spectrum $C_1$ in the frequency band U through a regression analysis.

The attenuation factor setting unit 334 then sets, as a predetermined initial value $\alpha_0$, the value of the attenuation factor candidate value $\alpha$ that is applied upon the performance of attenuation correction described below (Step S7). It is preferable that the initial value $\alpha_0$ should be stored in the attenuation factor information storage unit 373 in advance.

Next, the attenuation factor setting unit 334 performs attenuation correction on the features obtained by approximating each frequency spectrum by the feature calculation unit 333, using $\alpha$ as the attenuation factor candidate value, to calculate preliminarily corrected features, and stores the preliminarily corrected features together with the attenuation factor candidate value $\alpha$ in the feature information storage unit 372 (Step S8). The straight line $L_1$ illustrated in FIG. 7 is an example of a straight line obtained by the attenuation factor setting unit 334 performing the attenuation correction process.

In Step S8, the attenuation factor setting unit 334 does the calculations by substituting the data position Z = $(f_{sp} / 2v_s)$Dn obtained using data arrangement of a sound ray of an ultrasound signal into the reception depth z in the above-mentioned equations (2) and (4). $f_{sp}$ is the sampling frequency of data, $v_s$ is the velocity of sound, D is the data step width, and n is the number of data steps from the first data of the sound ray up to the data position of an amplification data group as a process target. For example, assuming that the data sampling frequency $f_{sp}$ is 50 MHz; the velocity of sound $v_s$ is 1530 m/sec; and the step width D is 15 by adopting the data arrangement illustrated in FIG. 4, z = 0.2295 n(mm).

The attenuation factor setting unit 334 calculates the variance of one preliminarily corrected feature selected from the plurality of preliminarily corrected features obtained by the attenuation factor setting unit 334 performing attenuation correction on each frequency spectrum, associates the variance with the attenuation factor candidate value $\alpha$, and stores the variance in the feature information storage unit 372 (Step S9). For example, if the preliminarily corrected features are the slope a and the midband fit c, the attenuation factor setting unit 334 calculates the variance of one of the preliminarily corrected features a and c as described above. It is preferable, as described above, that the variance of the preliminarily corrected feature a in the region of interest and the computation-purpose region be applied if the feature image data generation unit 342 uses the corrected feature a(h) in the subsequent process to generate feature image data, and the variance of the preliminarily corrected feature c in the region of interest and the computation-purpose region be applied if the feature image data generation unit 342 uses the corrected feature c(h) to generate feature image data. The preliminary correction amount whose variance is to be calculated may be preset, or the user may set a desired preliminarily corrected feature through the input unit 35.

The attenuation factor setting unit 334 then increments the value of the attenuation factor candidate value $\alpha$ by $\Delta\alpha$ (Step S10), and compares the magnitudes of the incremented attenuation factor candidate value $\alpha$ and a predetermined maximum value $\alpha_{max}$ (Step S11). If the attenuation factor candidate value $\alpha$ is larger than the maximum value $\alpha_{max}$ as the result of the comparison in Step S11 (Step S11: Yes), the ultrasound observation apparatus 3 proceeds to Step S12. On the other hand, if the attenuation factor candidate value $\alpha$ is equal to or less than the maximum value $\alpha_{max}$ as the result of the comparison in Step S11 (Step S11: No), the ultrasound observation apparatus 3 returns to Step S8. In the first embodiment, the region of interest and the computation-purpose region are assumed to have the same increment amount $\Delta\alpha$ and maximum value $\alpha_{max}$. However, increment amounts and/or maximum values may be set individually for the region of interest and the computation-purpose region.

In Step S12, the attenuation factor setting unit 334 refers to the variance of the preliminarily corrected feature of each attenuation factor candidate value stored in the feature information storage unit 372, for each of the region of interest and the computation-purpose region, and sets an attenuation factor candidate value having a minimum variance as the optimum attenuation factor (Step S12) .

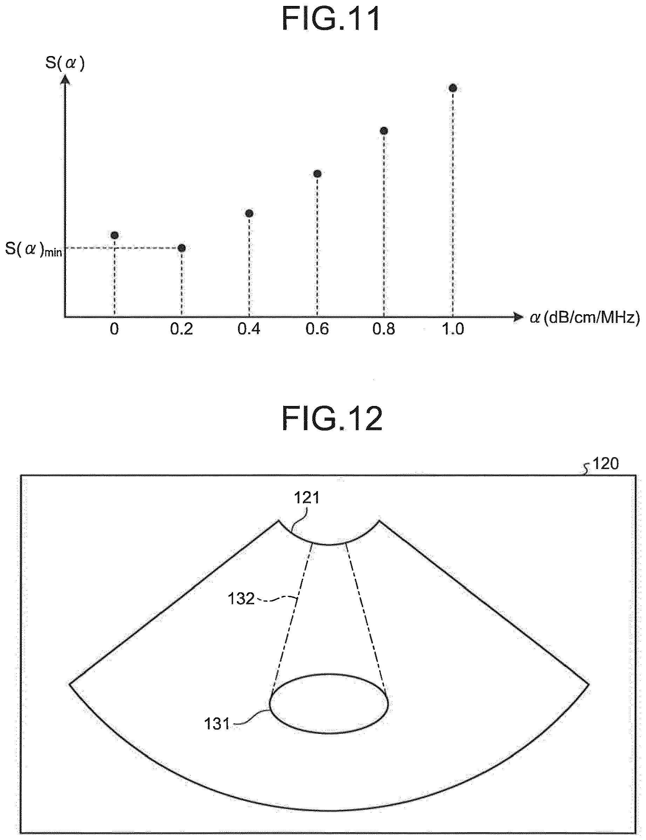

FIG. 11 is a diagram illustrating an overview of a process that is performed by the attenuation factor setting unit 334. FIG. 11 is a diagram illustrating an example of the relationship between the attenuation factor candidate value $\alpha$ and a variance $S(\alpha)$, where $\alpha_0$ = 0 (dB/cm/MHz), $\alpha_{max}$ = 1.0 (dB/cm/MHz), and $\Delta\alpha$ = 0.2 (dB/cm/MHz). In the case illustrated in FIG. 11, the variance takes a minimum value $S(\alpha)_{min}$ when the attenuation factor candidate value $\alpha$ is 0.2 (dB/cm/MHz). Therefore, in the case illustrated in FIG. 11, the attenuation factor setting unit 334 sets $\alpha$ = 0.2 (dB/cm/MHz) as the optimum attenuation factor.

The feature correction unit 335 then calculates the cumulative attenuation factor at the sampling point in the region of interest using the optimum attenuation factors $\alpha_{ROI}$ and $\alpha_c$ that are set respectively for the region of interest and the computation-purpose region by the attenuation factor setting unit 334 (Step S13). For example, the cumulative attenuation factor y(h) at the sampling point S(h) in the region of interest 111 illustrated in FIG. 6 is given by equation (7).

Next, the feature correction unit 335 performs attenuation correction on the features at the sampling point in the region of interest, using the cumulative attenuation factor, thereby to calculate corrected features (Step S14). For example, the feature correction unit 335 calculates the corrected features a(h), b(h), and c(h) of the slope $a_0$, the intercept $b_0$, and the midband fit $c_0$ at the sampling point S(h) in the region of interest 111 illustrated in FIG. 6 in accordance with equations (8) to (10), respectively.

The feature image data generation unit 342 superimposes visual information (for example, hue) associated with the corrected feature calculated in Step S14 on each pixel in the B-mode image data generated by the B-mode image data generation unit 341 to generate feature image data (Step S15). The feature image data generation unit 342 transmits the generated feature image data to the display device 4. The display device 4, which has received the feature image data, displays a feature image corresponding to the received feature image data.

After Step S15, the ultrasound observation apparatus 3 ends a series of the processing steps. The ultrasound observation apparatus 3 repeatedly and periodically executes the processing of Steps S1 to S15.

The computations of Steps S4 to S14 by the computing unit 33 may be performed using data after the coordinate conversion performed by the B-mode image data generation unit 341, or may be performed with RAW data before the coordinate conversion. While the accuracy of a feature can be further improved if the data after the coordinate conversion is used, the processing is performed in a state where the data is not interpolated if the data before the coordinate conversion is used; accordingly, it is possible to increase the computation speed.

According to the first embodiment of the present invention described above, an attenuation factor is set for the computation-purpose region being a region that is different from the region of interest in an ultrasound image, the region being used for computation for correcting features. Attenuation correction is performed on the features using the setting result to calculate corrected features at a sampling point in the region of interest in the ultrasound image. Hence, even if the observation target has non-uniform attenuation factors, the corrected features that take the non-uniformity into account can be calculated. Therefore, according to the first embodiment, it becomes possible to accurately identify the tissue characteristics of the observation target having non-uniform attenuation factors.

Moreover, according to the first embodiment, an attenuation factor is set for the region of interest in the ultrasound image. The cumulative attenuation factor per unit frequency at a sampling point in the region of interest is calculated based on the attenuation factors of the region of interest and the computation-purpose region. The cumulative attenuation factor is used to perform attenuation correction on the features to calculate corrected features at the sampling point. Accordingly, it is possible to appropriately set an attenuation factor in the middle between the surface of the ultrasound transducer and the sampling point, and it becomes possible to more accurately identify the tissue characteristics of the observation target having non-uniform attenuation factors.

(Modification 1-1)

FIG. 12 is a diagram schematically illustrating an example of the setting of a region of interest and a computation-purpose region in Modification 1-1 of the first embodiment. A region of interest 131 set in an ultrasound image 120 illustrated in FIG. 12 has an elliptical shape. A computation-purpose region 132 touches a surface position 121 of the ultrasound transducer 21 and the region of interest 131. Hence, a border of the computation-purpose region 132 with the region of interest 131 has an elliptical arc shape dished in a direction toward a smaller depth.

The region of interest may have a shape other than the elliptical arc shape, for example, a circular or trapezoidal shape. Also in this case, it is needless to say that the computation-purpose region has a shape in accordance with the shape of the region of interest.

(Modification 1-2)

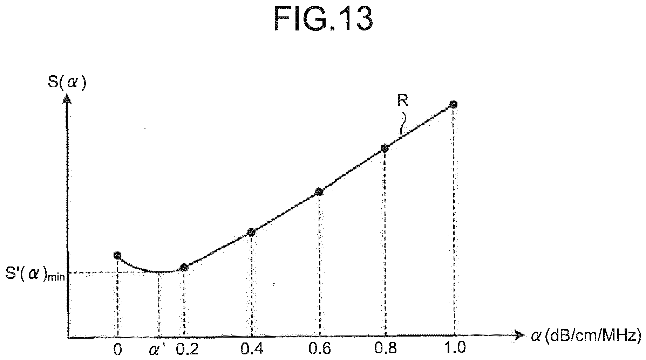

FIG. 13 is a diagram illustrating an overview of another method for the setting of an optimum attenuation factor that is performed by the attenuation factor setting unit 334 in Modification 1-2 of the first embodiment. FIG. 13 illustrates an example of the relationship between the attenuation factor candidate value $\alpha$ and the variance $S(\alpha)$, where $\alpha_0 = 0$ (dB/cm/MHz), $\alpha_{max} = 1.0$ (dB/cm/MHz) , and $\Delta\alpha = 0.2$ (dB/cm/MHz). The values of the variance $S(\alpha)$ for the attenuation factor candidate values $\alpha$ = 0, 0.2, 0.4, 0.6, 0.8, and 1.0 (all in dB/cm/MHz) are respectively the same as those in FIG. 11. In Modification 1-2, the feature calculation unit 333 performs a regression analysis before the attenuation factor setting unit 334 sets the optimum attenuation factor. Accordingly, a curve R that interpolates the values of the variance $S(\alpha)$ for the attenuation factor candidate value $\alpha$ is calculated. The attenuation factor setting unit 334 then calculates a minimum value $S'(\alpha)_{min}$ when 0 (dB/cm/MHz) $\le \alpha \le$ 1.0 (dB/cm/MHz) for the curve R, and sets a value $\alpha'$ of its attenuation factor candidate value as the optimum attenuation factor. Therefore, in Modification 1-2, the optimum attenuation factor $\alpha'$ is a value between 0 (dB/cm/MHz) and 0.2 (dB/cm/MHz).

(Second Embodiment)

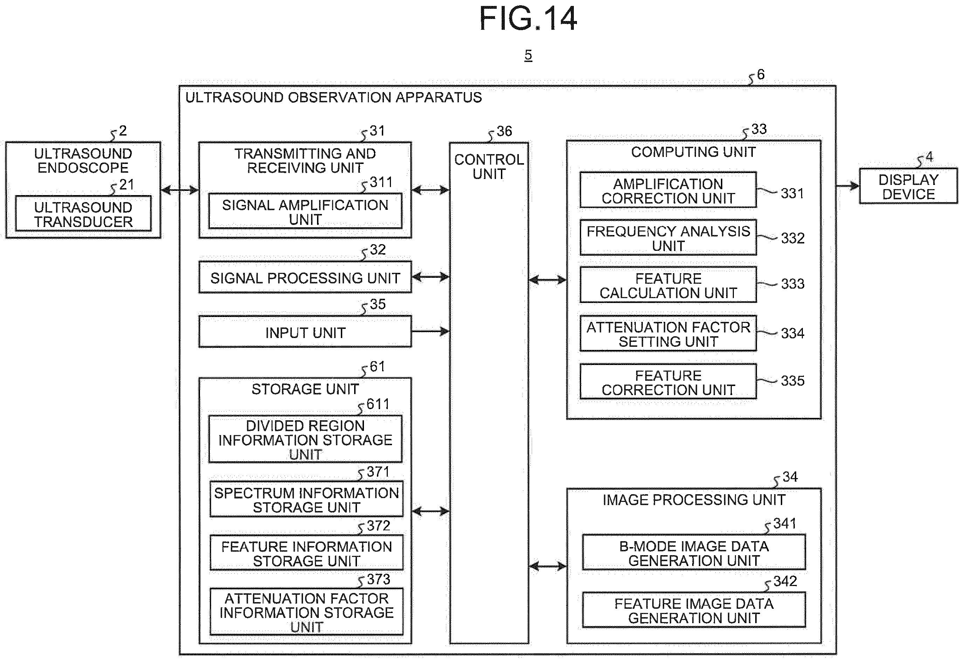

FIG. 14 is a block diagram illustrating a functional configuration of an ultrasound diagnosis system including an ultrasound observation apparatus according to a second embodiment of the present invention. An ultrasound diagnosis system 5 illustrated in FIG. 14 includes the ultrasound endoscope 2, an ultrasound observation apparatus 6, and the display device 4. In FIG. 14, the same reference signs as those of the ultrasound diagnosis system 1 described in the first embodiment are assigned to similar configurations to those of the ultrasound diagnosis system 1.

The ultrasound observation apparatus 6 includes the transmitting and receiving unit 31, the signal processing unit 32, the computing unit 33, the image processing unit 34, the control unit 36, and a storage unit 61. The storage unit 61 includes a divided region information storage unit 611 that stores information on divided regions obtained by dividing the computation-purpose region, in addition to the spectrum information storage unit 371, the feature information storage unit 372, and the attenuation factor information storage unit 373.

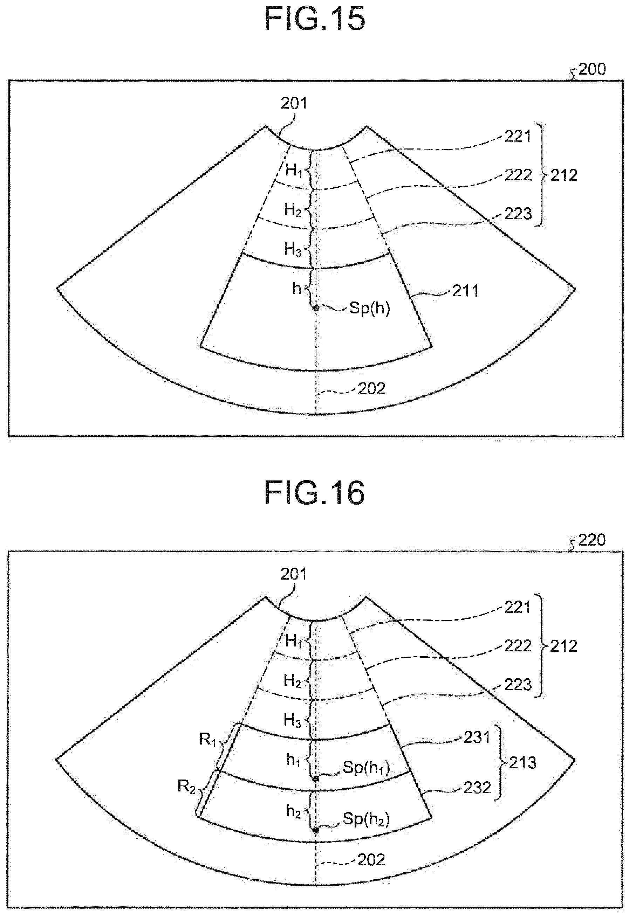

FIG. 15 is a diagram schematically illustrating an example of the setting of a region of interest and a computation-purpose region in an ultrasound image in the second embodiment. An ultrasound image 200 illustrated in FIG. 15 includes a region of interest 211 and a computation-purpose region 212. The region of interest 211 is a region surrounded by a total of four borders of two borders extending in a straight line from a surface position 201 of the ultrasound transducer 21 along the depth direction, and two borders having an arc shape along the scan angle direction. The computation-purpose region 212 touches the surface position 201 and the region of interest 211. Moreover, among borders of the computation-purpose region 212, two borders extending in a straight line from the surface position 201 along the depth direction are included in the same straight lines of the two borders of the region of interest 211 extending in a straight line from the surface position 201 along the depth direction. The computation-purpose region 212 includes divided regions 221, 222, and 223 obtained by dividing the computation-purpose region 212 into three along the depth direction. The widths (heights) in the depth direction of the divided regions 221, 222, and 223 are $H_1$, $H_2$, and $H_3$, respectively. The heights $H_1$, $H_2$, and $H_3$ can be set independently of one another. Also in the second embodiment, it is simply required to display at least the region of interest 211 when the display device 4 displays the ultrasound image 200.

In the case illustrated in FIG. 15, the cumulative attenuation factor y(h) at the sampling point Sp(h) whose distance in the depth direction from the border between the region of interest 211 and the computation-purpose region 212 is h in the region of interest 211, the sampling point being on a sound ray 202, is expressed as:

$$\gamma(h) \; = \; [\Sigma_{j=1,2,3} 2H_j \cdot \alpha_c(j)] \; + \; 2h \cdot \alpha_{ROI} \qquad \ldots \; (11).$$

Here, $\Sigma_{j=1,2,3}$ on the right-hand side indicates the sum of j = 1 to 3. $2H_j$ and $\alpha_c(j)$ of the first term on the right-hand side represent a round-trip distance of ultrasound and an optimum attenuation factor in the j-th divided region from the surface position 201 of the ultrasound transducer 21. In the case of FIG. 15, j = 1 corresponds to the divided region 221, j = 2 to the divided region 222, and j = 3 to the divided region 223. More generally, if the computation-purpose region 212 is divided into J, a calculating is made setting the sum of the first term on the right-hand side of equation (11) as j = 1- J.

Information related to the divided regions may be stored in advance in the divided region information storage unit 611. Alternatively, the information related to the divided regions may be stored in the divided region information storage unit 611 by the user setting the divided regions through the input unit 35.

When the computation-purpose region is divided, it is more preferable to make a division such that the area of a divided region is increased with increasing distance from the ultrasound transducer 21. Consequently, the S/N ratio in the distance can be improved.

When calculating corrected features at a sampling point in the region of interest, the ultrasound observation apparatus 6 sets optimum attenuation factors respectively for the region of interest and the divided regions, calculates a cumulative attenuation factor per unit frequency at the sampling point in the region of interest, using the optimum attenuation factors, performs attenuation correction on features using the cumulative attenuation factor, and calculates the corrected features. A method for setting an optimum attenuation factor for each divided region is similar to the method for setting optimum attenuation factors in the region of interest and the computation-purpose region, which is described in the first embodiment.

According to the second embodiment of the present invention described above, the computation-purpose region is divided into a plurality of divided regions. Hence, even if the observation target has non-uniform attenuation factors in the computation-purpose region, the corrected features that take the non-uniformity into account can be calculated. Therefore, according to the second embodiment, it becomes possible to accurately identify the tissue characteristics of the observation target having non-uniform attenuation factors as in the first embodiment.

(Modification 2-1)

FIG. 16 is a diagram schematically illustrating an example of the setting of a region of interest and a computation-purpose region in an ultrasound image in Modification 2-1 of the second embodiment. In Modification 2-1, in addition to the computation-purpose region 212, a region of interest 213 is divided into two divided regions 231 and 232 in an ultrasound image 220 illustrated in FIG. 16. The widths (heights) in the depth direction of the divided regions 231 and 232 are $R_1$ and $R_2$, respectively. The heights $R_1$ and $R_2$ can be set independently of one another. In this case, a cumulative attenuation factor $\gamma(h_1)$ at a sampling point $Sp(h_1)$ whose distance in the depth direction from a border, which is on a side close to the ultrasound transducer 21, of the divided region 231 is $h_1$, the sampling point being on the sound ray 202 in the divided region 231, is given by:

$$\gamma(h_1) = [\Sigma_{j=1,2,3}2H_j \cdot \alpha_C(j)] + 2h_1 \cdot \alpha_{ROI}(1) \ldots (12).$$

Here, $\alpha_{ROI}(1)$ of the second term on the right-hand side is the optimum attenuation factor in the first divided region 231 in increasing order of depth in the region of interest 213. Moreover, a cumulative attenuation factor $\gamma(h_2)$ at a sampling point $Sp(h_2)$ included in the divided region 232 is given by:

$$\gamma(h_2) = [\Sigma_{j=1,2,3}2H_j \cdot \alpha_C(j)] + 2R_1 \cdot \alpha_{ROI}(1) + 2h_2 \cdot \alpha_{ROI}(2) \ldots$$

$$(13).$$

Here, $2R_1$ of the second term on the right-hand side is a round-trip distance in the divided region 231. $\alpha_{ROI}(2)$ of the third term on the right-hand-side is the optimum attenuation factor of the divided region 232 whose depth is the second smallest in the region of interest 213.

More generally, a cumulative attenuation factor $\gamma(h_K)$ at a sampling point $h_K$ included in the K-th divided region in increasing order of depth in the region of interest is given by:

$$\gamma(h_K) = [\Sigma_{j=1,\ldots,J}2H_j \cdot \alpha_C(j)] + [\Sigma_{k=1,\ldots,k-1}2R_k \cdot \alpha_{ROI}(k)] + 2h_K \cdot$$

$$\alpha_{ROI}(K) \ldots (14)$$

When calculating corrected features at a sampling point in the region of interest, the ultrasound observation apparatus 6 sets optimum attenuation factors respectively for the divided regions in the region of interest and the computation-purpose region, calculates a cumulative attenuation factor per unit frequency at the sampling point in the region of interest, using the optimum attenuation factors, performs attenuation correction on features using the cumulative attenuation factor, and accordingly calculates the corrected features.

According to Modification 2-1 of the second embodiment described above, when tissue in the region of interest is non-uniform, it becomes possible to more accurately identify the tissue characteristics of the observation target.

In Modification 2-1, upon dividing the region of interest, it is more preferable to make the division such that the area of a divided region is increased with increasing distance from the ultrasound transducer 21 for the purpose of improving the S-N ratio in the distance.

Moreover, in Modification 2-1, divided regions may be set only in the region of interest, and the processing is performed assuming the computation-purpose region as one region as in the first embodiment.

(Third Embodiment)

A third embodiment of the present invention is characterized in that an attenuation factor of a computation-purpose region is set to be a predetermined constant. The configuration of an ultrasound system according to the third embodiment is similar to that of the ultrasound diagnosis system 1 described in the first embodiment.

In the third embodiment, a cumulative attenuation factor $\gamma'(h)$ at the sampling point $Sp(h)$ whose distance from the border on a side close to the ultrasound transducer 21 is h in the region of interest 111, the sampling point on the sound ray 102 illustrated in FIG. 6, is given by:

$$\gamma'(h) = 2H \cdot \alpha_C' + 2h \cdot \alpha_{ROI} \ldots (15).$$

Here, $\alpha_C'$ of the first term on the right-hand side is the attenuation factor of the computation-purpose region 112 and is a predetermined constant.

According to the third embodiment of the present invention described above, it becomes possible to more accurately identify tissue characteristics when it is preferable to have a constant attenuation factor regardless of the depth, depending on the observation target, because of the uniform computation-purpose region.

It may be preferable to make the attenuation factor of the region of interest constant, depending on the observation target. In such a case, it may be configured such that the optimum attenuation factor is calculated for the computation-purpose region while the attenuation factor for the region of interest is made constant.

Moreover, it may be preferable to make both of the attenuation factors of the region of interest and the computation-purpose region constant, depending on the observation target. In such a case, the attenuation factors of the region of interest and the computation-purpose region may be made constant.

(Other Embodiments)

The modes for carrying out the present invention have been described so far. However, the present invention is not intended to be limited only to the above-mentioned first to third embodiments. For example, it may be configured to be able to select the on/off of the mode that uses the computation-purpose region with an input from the input unit 35. With such a configuration, in a mode that does not use the computation-purpose region, it becomes possible to perform processing at high speed with a little amount of calculation of the computing unit 33; accordingly, it is possible to increase the frame rate.

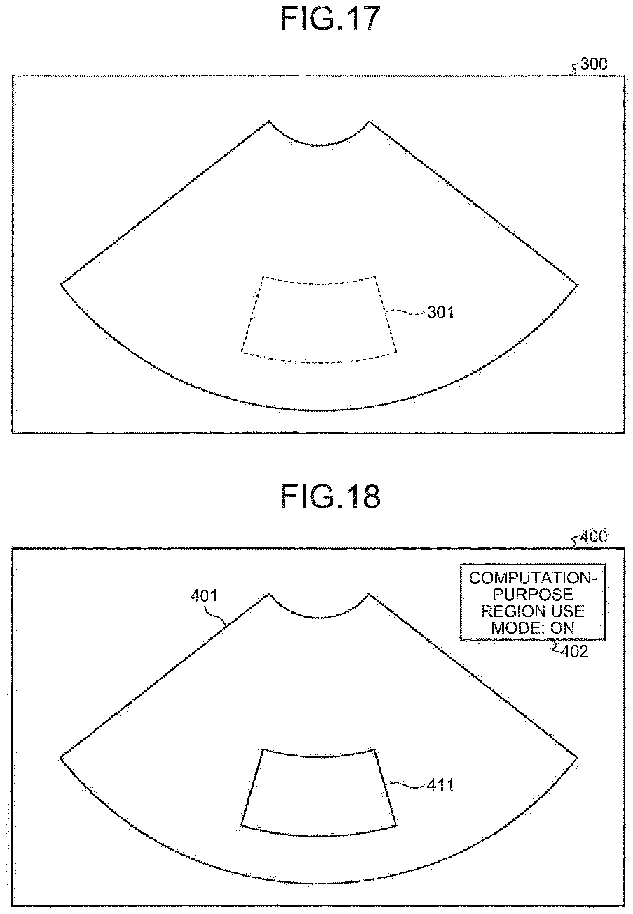

Moreover, the display mode of the display device 4 may be changed according to the on/off of the mode that uses the computation-purpose region. FIG. 17 is a diagram schematically illustrating an example of the display of a region of interest on the display device 4 of when the mode that does not use the computation-purpose region is set. An ultrasound image 300 illustrated in FIG. 17 displays a region of interest 301 by broken lines. In contrast, the region of interest 111 in the ultrasound image 100 illustrated in FIG. 6 is displayed by the solid lines, and corresponds to a case where the mode that uses the computation-purpose region 112 is set. In this manner, the mode of display of the region of interest on the display device 4 is changed according to the on/off of the mode that uses the computation-purpose region; accordingly, the user can intuitively grasp the set processing mode. The mode of display may be changed by changing the color, weight of a line, and the like of the region of interest. Moreover, when the mode that uses the computation-purpose region is set, the display device 4 may display the computation-purpose region in the ultrasound image in a mode that is distinguishable from the region of interest.

FIG. 18 is a diagram illustrating another example of the display of a region of interest on the display device 4 of when the mode that uses the computation-purpose region is set. An ultrasound image 400 illustrated in FIG. 18 includes an ultrasound image display section 401 and a mode display section 402. A region of interest 411 is displayed in the ultrasound image display section 401. "Computation-purpose region use mode: on" is described in the mode display section 402. In contrast, when the mode that uses the computation-purpose region is not set, "computation-purpose region use mode: off" may be displayed in the mode display section 402, or the mode display section 402 itself may not be displayed. Moreover, the mode display section 402 may simply display "on" or "off" according to the on or off of the mode that uses the computation-purpose region. Also in the case described here, the user can intuitively grasp the set processing mode.

Moreover, the attenuation factor setting unit 334 may calculate an equivalent optimum attenuation factor value corresponding to an optimum attenuation factor in all frames of the ultrasound image, and set, as the optimum attenuation factor, the mean, median, or mode of a predetermined number of equivalent optimum attenuation factor values including an equivalent optimum attenuation factor value of the latest frame. In this case, the number of changes in optimum attenuation factor is reduced as compared to a case of setting an optimum attenuation factor for each frame, so that the value can be stabilized.

Moreover, the attenuation factor setting unit 334 may set an optimum attenuation factor in predetermined frame intervals of the ultrasound image. Consequently, the amount of calculation can be significantly reduced. In this case, it is simply required to use an optimum attenuation factor value that was set last until the next optimum attenuation factor is set.

Moreover, the input unit 35 may receive the input of a change in the setting of the initial value $\alpha_0$ of the attenuation factor candidate value.

Moreover, it is also possible to apply any of, for example, the standard deviation, the difference between the maximum value and the minimum value of a feature in a population, and the half width of the distribution of features, as the quantity that gives statistical dispersion. A case where the inverse of a variance is applied as the quantity that gives statistical dispersion is also conceivable. In this case, however, it is needless to say that an attenuation factor candidate value having a maximum value is the optimum attenuation factor.

Moreover, it is also possible that the attenuation factor setting unit 334 calculates the statistical dispersion of a plurality of kinds of preliminarily corrected features, and set, as the optimum attenuation factor, an attenuation factor candidate value having minimal statistical dispersion.

Moreover, the attenuation factor setting unit 334 may calculate a preliminarily corrected feature by performing attenuation correction on a frequency spectrum with a plurality of attenuation factor candidate values, and performing a regression analysis on the frequency spectrum after attenuation correction.

Moreover, an application to an ultrasound probe, other than an ultrasound endoscope, is also possible. For example, a slim ultrasound miniature probe without an optical system may be applied as the ultrasound probe. The ultrasound miniature probe is generally used to be inserted into the biliary tract, bile duct, pancreatic duct, trachea, bronchus, urethra, or ureter and observe its surrounding organ (such as the pancreas, lung, prostate, bladder, or lymph node). Moreover, an external ultrasound probe that applies ultrasound from the body surface of a subject may be applied as the ultrasound probe. The external ultrasound probe is generally used to observe an abdominal organ (the liver, gallbladder, or bladder), the breast (especially, the mammary gland), and the thyroid.

In this manner, the present invention can include various embodiments within the range that does not depart from the technical idea described in the claims.

Industrial Applicability

As described above, an ultrasound observation apparatus, a method for operating the ultrasound observation apparatus, and a program for operating the ultrasound observation apparatus according to the present invention are useful for accurately identifying tissue characteristics of an observation target having non-uniform attenuation factors.

Reference Signs List

| | |
|---|---|
| 1, 5 | Ultrasound diagnosis system |
| 2 | Ultrasound endoscope |
| 3, 6 | Ultrasound observation apparatus |
| 4 | Display device |
| 21 | Ultrasound transducer |
| 31 | Transmitting and receiving unit |
| 32 | Signal processing unit |
| 33 | Computing unit |
| 34 | Image processing unit |
| 35 | Input unit |
| 36 | Control unit |
| 37, 61 | Storage unit |
| 100, 120, 200, 220, 300, 400 | Ultrasound image |
| 101, 121 | Surface position |
| 111, 131, 211, 213, 301, 411 | Region of interest |
| 112, 132, 212 | Computation-purpose region |
| 221, 222, 223, 231, 232 | Divided region |
| 311 | Signal amplification unit |
| 331 | Amplification correction unit |
| 332 | Frequency analysis unit |
| 333 | Feature calculation unit |
| 334 | Attenuation factor setting unit |
| 335 | Feature correction unit |
| 341 | B-mode image data generation unit |
| 342 | Feature image data generation unit |
| 371 | Spectrum information storage unit |
| 372 | Feature information storage unit |
| 373 | Attenuation factor information storage unit |
| 401 | Ultrasound image display section |
| 402 | Mode display section |
| 611 | Divided region information storage unit |

**Claims**

1. An ultrasound observation apparatus for generating an ultrasound image based on an ultrasound signal acquired by an ultrasound probe, the ultrasound probe having an ultrasound transducer configured to transmit ultrasound to an observation target and to receive the ultrasound reflected from the observation target, the ultrasound observation apparatus comprising:

a frequency analysis unit configured to analyze a frequency of the ultrasound signal to calculate a plurality of frequency spectra in accordance with reception depths and receiving directions of the ultrasound signal;
a feature calculation unit configured to calculate a feature of each of the plurality of frequency spectra;
an attenuation factor setting unit configured to set an attenuation factor of a computation-purpose region, the computation-purpose region being a region that is different from a region of interest in the ultrasound image and is used for computation for correcting the feature; and
a feature correction unit configured to perform attenuation correction on the feature using the attenuation factor set by the attenuation factor setting unit, thereby to calculate a corrected feature at a sampling point in the region of interest in the ultrasound image.

2. The ultrasound observation apparatus according to claim 1, wherein
the attenuation factor setting unit is configured to set an attenuation factor of the region of interest in the ultrasound image, and
the feature correction unit is configured to:

calculate a cumulative attenuation factor per unit frequency at the sampling point based on the attenuation factor of the region of interest and the attenuation factor of the computation-purpose region, set by the attenuation factor setting unit; and
perform the attenuation correction on the feature using the cumulative attenuation factor, thereby to calculate the corrected feature at the sampling point.

3. The ultrasound observation apparatus according to claim 2, wherein
the computation-purpose region is a region located between a surface of the ultrasound transducer and the region of interest.

4. The ultrasound observation apparatus according to claim 1, wherein
the attenuation factor setting unit is configured to:

use each of a plurality of attenuation factor candidate values per unit length and per unit frequency that give different attenuation characteristics when the ultrasound propagates through the observation target, to perform attenuation correction on the feature of each of the frequency spectra for removing an influence of the ultrasound, and thereby to calculate a preliminarily corrected feature of each of the frequency spectra for each of the attenuation factor candidate values; and
set an optimum attenuation factor for the observation target from among the plurality of attenuation factor candidate values based on a result of calculating the preliminarily corrected feature.

5. The ultrasound observation apparatus according to claim 1, wherein
the attenuation factor setting unit is configured to divide the computation-purpose region into a plurality of divided regions, and to set the attenuation factor for each of the divided regions.

6. The ultrasound observation apparatus according to claim 2, wherein
the attenuation factor setting unit is configured to divide the region of interest into a plurality of divided regions, and to set the attenuation factor for each of the divided regions.

7. The ultrasound observation apparatus according to claim 1, wherein
the attenuation factor setting unit is configured to set a constant as the attenuation factor of the computation-purpose region.

8. The ultrasound observation apparatus according to claim 7, wherein
the attenuation factor setting unit is configured to:

use, as an attenuation factor of the region of interest, each of a plurality of attenuation factor candidate values per unit length and per unit frequency that give different attenuation characteristics when the ultrasound propagates through the observation target, to perform attenuation correction on the feature of each of the frequency spectra for removing an influence of the ultrasound, and thereby to calculate a preliminarily corrected feature of each of the frequency spectra for each of the attenuation factor candidate values; and
set an optimum attenuation factor for the observation target from among the plurality of attenuation factor candidate values based on a result of calculating the preliminarily corrected feature.

**9.** The ultrasound observation apparatus according to claim 2, wherein
the attenuation factor setting unit is configured to set a predetermined fixed value as the attenuation factor of the region of interest.

**10.** The ultrasound observation apparatus according to any one of claims 7 to 9, further comprising an input unit configured to receive input of information for setting the attenuation factor, wherein
the attenuation factor setting unit is configured to set the attenuation factor based on the information received by the input unit.

**11.** The ultrasound observation apparatus according to claim 5 or 6, wherein
in two adjacent divided regions of the plurality of divided regions along a depth direction, one of the two adjacent divided regions located more distant from the ultrasound transducer has a length in the depth direction equal to or more than a length in the depth direction of the other of the two adjacent divided regions located closer to the ultrasound transducer.

**12.** The ultrasound observation apparatus according to claim 4, wherein
the attenuation factor setting unit is configured to calculate a statistical dispersion of the preliminarily corrected feature for each of the attenuation factor candidate values, and to set an attenuation factor candidate value having a minimal statistical dispersion to be the optimum attenuation factor.

**13.** The ultrasound observation apparatus according to claim 1, further comprising a feature image data generation unit configured to generate feature image data for showing information on the corrected feature together with the ultrasound image.

**14.** A method for operating an ultrasound observation apparatus for generating an ultrasound image based on an ultrasound signal acquired by an ultrasound probe, the ultrasound probe having an ultrasound transducer configured to transmit ultrasound to an observation target and to receive the ultrasound reflected from the observation target, the method comprising:

a frequency analysis step of analyzing, by a frequency analysis unit, a frequency of the ultrasound signal to calculate a plurality of frequency spectra in accordance with reception depths and receiving directions of the ultrasound signal;
a feature calculation step of calculating, by a feature calculation unit, a feature of each of the plurality of frequency spectra;
an attenuation factor setting step of setting, by an attenuation factor setting unit, an attenuation factor of a computation-purpose region, the computation-purpose region being a region that is different from a region of interest in the ultrasound image and is used for computation for correcting the feature; and
a feature correction step of performing, by a feature correction unit, attenuation correction on the feature using the attenuation factor set in the attenuation factor setting step, thereby to calculate a corrected feature at a sampling point in the region of interest in the ultrasound image.

**15.** A program for operating an ultrasound observation apparatus for generating an ultrasound image based on an ultrasound signal acquired by an ultrasound probe, the ultrasound probe having an ultrasound transducer configured to transmit ultrasound to an observation target and to receive the ultrasound reflected from the observation target, the program causing the ultrasound observation apparatus to execute:

a frequency analysis step of analyzing, by a frequency analysis unit, a frequency of the ultrasound signal to calculate a plurality of frequency spectra in accordance with reception depths and receiving directions of the ultrasound signal;
a feature calculation step of calculating, by a feature calculation unit, a feature of each of the plurality of frequency spectra;
an attenuation factor setting step of setting, by an attenuation factor setting unit, an attenuation factor of a computation-purpose region, the computation-purpose region being a region that is different from a region of interest in the ultrasound image and is used for computation for correcting the feature; and
a feature correction step of performing, by a feature correction unit, attenuation correction on the feature using the attenuation factor set in the attenuation factor setting step, thereby to calculate a corrected feature at a sampling point in the region of interest in the ultrasound image.

# FIG.1

<u>1</u>

ULTRASOUND OBSERVATION APPARATUS ⌐3

ULTRASOUND ENDOSCOPE ⌐2

ULTRASOUND TRANSDUCER ⌐21

TRANSMITTING AND RECEIVING UNIT ⌐31

SIGNAL AMPLIFICATION UNIT ⌐311

SIGNAL PROCESSING UNIT ⌐32

INPUT UNIT ⌐35

STORAGE UNIT ⌐37

SPECTRUM INFORMATION STORAGE UNIT ⌐371

FEATURE INFORMATION STORAGE UNIT ⌐372

ATTENUATION FACTOR INFORMATION STORAGE UNIT ⌐373

CONTROL UNIT ⌐36

COMPUTING UNIT ⌐33

AMPLIFICATION CORRECTION UNIT ～331

FREQUENCY ANALYSIS UNIT ～332

FEATURE CALCULATION UNIT ～333

ATTENUATION FACTOR SETTING UNIT ～334

FEATURE CORRECTION UNIT ～335

IMAGE PROCESSING UNIT ⌐34

B-MODE IMAGE DATA GENERATION UNIT ⌐341

FEATURE IMAGE DATA GENERATION UNIT ⌐342

DISPLAY DEVICE ⌐4

EP 3 275 376 A1

# FIG.2

# FIG.3

FIG.4

# FIG.5

# FIG.6

# FIG.7

# FIG.8

# FIG.9

```
                    START

RECEIVE MEASUREMENT RESULT OF OBSERVATION        S1
              TARGET

AMPLIFY RECEIVED ECHO SIGNAL (STC CORRECTION)    S2

        GENERATE B-MODE IMAGE DATA               S3

            CORRECT AMPLIFICATION                S4

              FREQUENCY ANALYSIS                 S5

    CALCULATE FEATURES AT SAMPLING POINTS IN
  REGION OF INTEREST AND COMPUTATION-PURPOSE     S6
                  REGION
```

$\alpha = \alpha_0$  — S7

PERFORM ATTENUATION CORRECTION ON FEATURES
FOR EACH FREQUENCY SPECTRUM USING $\alpha$ AS — S8
ATTENUATION FACTOR CANDIDATE VALUE

CALCULATE VARIANCE OF PRELIMINARILY CORRECTED
FEATURE AFTER ATTENUATION CORRECTION FOR — S9
EACH OF REGION OF INTEREST AND COMPUTATION-
PURPOSE REGION

$\alpha = \alpha + \Delta\alpha$ — S10

S11

$\alpha > \alpha_{max}$?  NO

YES

SET, AS OPTIMUM ATTENUATION FACTOR,
ATTENUATION FACTOR CANDIDATE VALUE HAVING
MINIMUM VARIANCE OF PRELIMINARILY CORRECTED — S12
FEATURE IN EACH OF REGION OF INTEREST AND
COMPUTATION-PURPOSE REGION

CALCULATE CUMULATIVE ATTENUATION FACTOR AT — S13
SAMPLING POINT IN REGION OF INTEREST

PERFORM ATTENUATION CORRECTION ON FEATURES
AT SAMPLING POINT IN REGION OF INTEREST USING — S14
CUMULATIVE ATTENUATION FACTOR

GENERATE FEATURE IMAGE DATA — S15

END

# FIG.10

```
        ┌─────────────────┐
        │   FREQUENCY     │
        │   ANALYSIS      │
        └────────┬────────┘
                 ↓
        ┌─────────────────┐
        │     k=k₀        │  ～S21
        └────────┬────────┘
                 ↓
        ┌─────────────────┐
        │  Z⁽ᵏ⁾=Z⁽ᵏ⁾₀    │  ～S22
        └────────┬────────┘
                 ↓
    ┌────────────────────────┐
    │ ACQUIRE SAMPLE DATA GROUP│  ～S23
    └────────────┬───────────┘
                 ↓
    ┌────────────────────────┐
    │  APPLY WINDOW FUNCTION  │  ～S24
    └────────────┬───────────┘
                 ↓
         ／＼  ～S25
        IS SAMPLE DATA      NO
        GROUP NORMAL?  ──────────→  ┌──────────────────┐  S26
         ＼／                        │ INSERT ZERO DATA │
          │ YES                     │   TO COVER       │
          │                         │   SHORTFALL      │
          │                         └──────────────────┘
          ↓                                  │
    ┌────────────────────────┐               │
    │   FFT COMPUTATION       │  ～S27 ←──────┘
    └────────────┬───────────┘
                 ↓
    ┌────────────────────────┐
    │    Z⁽ᵏ⁾=Z⁽ᵏ⁾+D        │  ～S28
    └────────────┬───────────┘
                 ↓
          ／＼  ～S29
      Z⁽ᵏ⁾>Z⁽ᵏ⁾ₘₐₓ?  ────── NO
          ＼／
          │ YES
          ↓
    ┌────────────────────────┐
    │       k=k+1            │  ～S30
    └────────────┬───────────┘
                 ↓
    NO    ／＼  ～S31
    ←──  k>kₘₐₓ?
          ＼／
          │ YES
          ↓
     ┌──────────┐
     │  RETURN  │
     └──────────┘
```

$k = k_0$ ～S21

$Z^{(k)} = Z^{(k)}_0$ ～S22

ACQUIRE SAMPLE DATA GROUP ～S23

APPLY WINDOW FUNCTION ～S24

IS SAMPLE DATA GROUP NORMAL? ～S25

INSERT ZERO DATA TO COVER SHORTFALL S26

FFT COMPUTATION ～S27

$Z^{(k)} = Z^{(k)} + D$ ～S28

$Z^{(k)} > Z^{(k)}_{max}?$ ～S29

$k = k+1$ ～S30

$k > k_{max}?$ ～S31

# FIG.11

# FIG.12

# FIG.13

# FIG.14

ULTRASOUND OBSERVATION APPARATUS — 6

- ULTRASOUND ENDOSCOPE — 2
  - ULTRASOUND TRANSDUCER — 21
- TRANSMITTING AND RECEIVING UNIT — 31
  - SIGNAL AMPLIFICATION UNIT — 311
- CONTROL UNIT — 36
- COMPUTING UNIT — 33
  - AMPLIFICATION CORRECTION UNIT — 331
  - FREQUENCY ANALYSIS UNIT — 332
  - FEATURE CALCULATION UNIT — 333
  - ATTENUATION FACTOR SETTING UNIT — 334
  - FEATURE CORRECTION UNIT — 335
- DISPLAY DEVICE — 4
- SIGNAL PROCESSING UNIT — 32
- INPUT UNIT — 35
- STORAGE UNIT — 61
  - DIVIDED REGION INFORMATION STORAGE UNIT — 611
  - SPECTRUM INFORMATION STORAGE UNIT — 371
  - FEATURE INFORMATION STORAGE UNIT — 372
  - ATTENUATION FACTOR INFORMATION STORAGE UNIT — 373
- IMAGE PROCESSING UNIT — 34
  - B-MODE IMAGE DATA GENERATION UNIT — 341
  - FEATURE IMAGE DATA GENERATION UNIT — 342

5

EP 3 275 376 A1

# FIG.15

# FIG.16

# FIG.17

300

301

# FIG.18

400

401

COMPUTATION-
PURPOSE
REGION USE
MODE: ON

402

411

## INTERNATIONAL SEARCH REPORT

| International application No. |
| --- |
| PCT/JP2015/083929 |

### A. CLASSIFICATION OF SUBJECT MATTER
*A61B8/14*(2006.01)i

According to International Patent Classification (IPC) or to both national classification and IPC

### B. FIELDS SEARCHED

Minimum documentation searched (classification system followed by classification symbols)
A61B8/14

Documentation searched other than minimum documentation to the extent that such documents are included in the fields searched

| | | | |
| --- | --- | --- | --- |
| Jitsuyo Shinan Koho | 1922–1996 | Jitsuyo Shinan Toroku Koho | 1996–2016 |
| Kokai Jitsuyo Shinan Koho | 1971–2016 | Toroku Jitsuyo Shinan Koho | 1994–2016 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| A | JP 5659324 B1 (Olympus Medical Systems Corp.), 28 January 2015 (28.01.2015), paragraphs [0022] to [0035], [0043] to [0049], [0068]; fig. 1 to 5, 10 to 12, 18 & US 2015/0178919 A1 & WO 2014/192954 A1 & CN 104582584 A | 1–15 |
| A | JP 5568199 B1 (Olympus Medical Systems Corp.), 06 August 2014 (06.08.2014), paragraphs [0028] to [0041], [0062], [0063], [0082], [0083], [0109], [0110] & US 2014/0309531 A1 & WO 2014/054469 A1 & EP 2904975 A1 & CN 104125804 A | 1–15 |

[×] Further documents are listed in the continuation of Box C.   [ ] See patent family annex.

| | |
| --- | --- |
| *   Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
| --- | --- |
| 22 January 2016 (22.01.16) | 02 February 2016 (02.02.16) |

| Name and mailing address of the ISA/ | Authorized officer |
| --- | --- |
| Japan Patent Office<br>3-4-3,Kasumigaseki,Chiyoda-ku,<br>Tokyo 100-8915,Japan | |
| | Telephone No. |

Form PCT/ISA/210 (second sheet) (January 2015)

**INTERNATIONAL SEARCH REPORT**

International application No.

PCT/JP2015/083929

C (Continuation).   DOCUMENTS CONSIDERED TO BE RELEVANT

| Category* | Citation of document, with indication, where appropriate, of the relevant passages | Relevant to claim No. |
|---|---|---|
| A | WO 2012/063976 A1  (Olympus Medical Systems Corp.), 18 May 2012 (18.05.2012), entire text; all drawings & JP 5054254 B         & US 2012/0310087 A1 & EP 2548512 A1         & CN 102802536 A | 1-15 |
| A | WO 2012/063929 A1  (Olympus Medical Systems Corp.), 18 May 2012 (18.05.2012), entire text; all drawings & JP 5307939 B         & US 2013/0030296 A1 & EP 2599441 A1         & CN 103153195 A | 1-15 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010246640 A